# EUROPEAN PATENT APPLICATION

(11) **EP 4 252 773 A1**
(43) Date of publication of application: **04.10.2023**
(21) Application number: 21898063.9
(22) Date of filing: 26.11.2021
(51) Int. Cl.: A61K 45/00, A61P 11/00, A61P 35/00, A61P 43/00, C12Q 1/686, G01N 33/68

(54) **CANCER EXAMINATION METHOD AND CANCER TREATMENT AGENT**

(30) Priority: 27.11.2020 JP 2020196899
(71) Applicant: Imamitsukai Medical Corporation, Kitakyushu-shi, Fukuoka 808-0071 (JP)
(72) Inventor: OYAMA Tsunehiro, Kitakyushu-shi, Fukuoka 808-0071 (JP)
(74) Representative: Hoffmann Eitle
(86) International application number: PCT/JP2021/043304
(87) International publication number: WO 2022/114091

(57) **Abstract**

The present invention relates to a method for predicting a prognosis of a lung adenocarcinoma patient. The present invention relates to a method for predicting a prognosis of a lung adenocarcinoma patient after cancer resection. The present invention also relates to a method for treating a lung adenocarcinoma patient.

## Description

### Technical Field

The present invention relates to a cancer examination method and a cancer treatment agent.

### Background Art

The number of deaths in Japan in 2018 was 1,360,000, of which 370,000 (27%) died of cancer. In addition, 980,000 people were newly diagnosed with cancer in 2018; thus, accurate diagnosis of cancer is a social task.

Non Patent Literature 1 discloses that examination of mRNA expressions of SGLT1 and SGLT2 found no difference between the expressions in lung cancer and those in normal lung tissues. It is disclosed that SGLT2 is highly expressed and plays a role in glucose uptake in metastasis. Non Patent Literature 2 discloses that evaluation of tissues containing much lung adenocarcinoma found high mRNA expression of SGLT2 in cancer patients. Non Patent Literature 3 discloses that lung cancer causes no difference in expression of SGLT1. Non Patent Literature 4 discloses that SGLT2 is highly expressed in early-stage/low-grade lung adenocarcinoma before malignant transformation.

### Citation List

### Non Patent Literature

Non Patent Literature 1: Ishikawa N. et al., Jpn. J. Cancer Res., 92, 874-879, 2001
Non Patent Literature 2: Zhang X., et al., International Journal of Oncology, 54, 199-208, 2019
Non Patent Literature 3: Madunic I. V. et al., Arh. Hig. Rada. Toksikol., 69, 278-285, 2018
Non Patent Literature 4: Scafoglio C. R. et al., Sci. Transl. Med., 10, 467, 2018

### Summary of Invention

The present invention provides a cancer examination method and a cancer treatment agent.

The present inventors have found that the prognoses of patients after cancer resection can be predicted on the basis of expressions of genes of the SGLT family in resected cancer tissues.

The present invention provides, for example, the followings.
[1] A method for predicting a prognosis of a cancer patient, the method including:
   (1) a detection step of detecting expression(s) of either one or both of SGLT1 and SGLT2 in a cancer tissue obtained from a cancer patient, wherein a case that the cancer tissue is positive for expression of SGLT1 indicates that the prognosis is good, and a case that the cancer tissue is positive for expression of SGLT2 indicates that the prognosis is poor, or
   (2) predicting that the prognosis is good if the patient is a diabetes mellitus patient, and predicting that the prognosis is poor if the patient is a non-diabetes mellitus patient.
[2] The method according to [1], wherein the cancer patient is a lung adenocarcinoma patient.
[3] The method according to [1] or [2], wherein the cancer patient is a patient after cancer resection.
[4] The method according to any one of [1] to [3], wherein the level of each expression is determined on the basis of the ratio of positive cells to cancer cells.
[5] The method according to any one of [1] to [4], wherein each expression is expression of protein.
[6] The method according to any one of [1] to [5], wherein the detection step includes detecting expression of SGLT1.
[7] The method according to any one of [1] to [6], wherein the detection step includes detecting expression of SGLT2.
[8] The method according to any one of [1] to [7], wherein the prognosis is based on at least one or more selected from the group consisting of the recurrence rate and the survival rate.
[9] The method according to any one of [1] to [8], including both of (1) and (2), and further including either one or both of:
   (a) predicting that the prognosis of the patient is good if the patient is a diabetes mellitus patient, and positive for expression of SGLT1 and/or negative for expression of SGLT2; and
   (b) predicting that the prognosis of the patient is poor if the patient is a non-diabetes mellitus patient, and negative for expression of SGLT1 and/or positive for expression of SGLT2.
[10] The method according to any one of [1] to [9], wherein the lung adenocarcinoma is early-stage cancer selected from the group consisting of Stage I cancer and Stage II cancer.
[11] A pharmaceutical composition containing an SGLT2 inhibitor for use in treating lung adenocarcinoma in a patient positive for expression of SGLT2.
[12] The pharmaceutical composition according to [11], wherein the patient is a patient predicted to have a poor prognosis by the method according to any one of [1] to [10] .
[13] The pharmaceutical composition according to [11], wherein the patient is a lung adenocarcinoma patient further having diabetes mellitus.
[14] The pharmaceutical composition according to [11] or [13], wherein the patient is further positive for expression of SGLT1.
[15] The pharmaceutical composition according to any one of [11] to [13], wherein the patient is negative for expression of SGLT1.

[1A] A method for predicting a prognosis of a cancer patient, the method including:
   a detection step of detecting expression of SGLT2 protein in a lung adenocarcinoma tissue obtained from a lung adenocarcinoma patient, wherein a case that the adenocarcinoma tissue is negative for expression of SGLT2 indicates that the prognosis is good, and a case that the adenocarcinoma tissue is positive for expression of SGLT2 indicates that the prognosis is poor.
[2A] The method according to [1A], wherein the cancer patient is a patient after cancer resection.
[3A] The method according to [1A] or [2A], wherein whether the cancer tissue is positive or negative for the expression is determined on the basis of the ratio of positive cells to cancer cells.
[4A] The method according to any one of [1A] to [3A], further including predicting that the prognosis is good if the patient has a mutation in epidermal growth factor receptor (EGFR) and predicting that the prognosis is poor if the patient has no mutation in EGFR.
[5A] The method according to any one of [1A] to [4A], wherein the prognosis is based on at least one or more selected from the group consisting of the recurrence rate and the survival rate.
[6A] The method according to any one of [1A] to [5A], further including determining whether the patient is a diabetes mellitus patient or not, wherein
   (a) a case that the patient is a diabetes mellitus patient and negative for expression of SGLT2 indicates that the prognosis of the patient is good, or
   (b) a case that the patient is a non-diabetes mellitus patient and positive for expression of SGLT2 indicates that the prognosis of the patient is poor.
[7A] The method according to any one of [1A] to [6A], wherein the lung adenocarcinoma is early-stage cancer selected from the group consisting of Stage I cancer and Stage II cancer.
[8A] A pharmaceutical composition containing an SGLT2 inhibitor for use in treating lung adenocarcinoma in an SGLT2-positive patient, wherein, in a preferred embodiment, the patient may be a patient predicted to have a poor prognosis by the method according to any one of [1A] to [7A].
[9A] The pharmaceutical composition according to [8A], wherein the patient is an SGLT2-positive diabetes mellitus patient.
[10A] The pharmaceutical composition according to [8A], wherein the patient is an SGLT2-positive non-diabetes mellitus patient.
[11A] A method for predicting a prognosis of a lung adenocarcinoma patient, the method including:
   a detection step of detecting expression of SGLT1 in a lung adenocarcinoma tissue obtained from a lung adenocarcinoma patient, wherein a case that the adenocarcinoma tissue is positive for expression of SGLT1 indicates that the prognosis is good or possibly good, and a case that the adenocarcinoma tissue is negative for expression of SGLT1 indicates that the prognosis is poor or possibly poor.
[12A] The method according to [11A], further including a detection step of detecting expression of SGLT2 in the lung adenocarcinoma tissue, wherein a case that the adenocarcinoma tissue is positive for expression of SGLT1 and negative for expression of SGLT2 indicates that the prognosis is good or possibly good, and a case that the adenocarcinoma tissue is negative for expression of SGLT1 and positive for expression of SGLT2 indicates that the prognosis is poor or possibly poor.
[13A] The method according to [11A] or [12A], wherein the adenocarcinoma patient is a patient after cancer resection.
[14A] The method according to any one of [11A] to [13A], wherein whether the adenocarcinoma tissue is positive or negative for each expression is determined on the basis of the ratio of positive cells to cancer cells.
[15A] The method according to any one of [11A] to [14A], wherein each expression is expression of protein.
[16A] The method according to any one of [11A] to [15A], wherein the prognosis is based on at least one or more selected from the group consisting of the recurrence rate and the survival rate.
[17A] The method according to any one of [11A] to [16A], further including determining whether the patient is a diabetes mellitus patient or not, wherein
   (a) a case that the patient is a diabetes mellitus patient and positive for expression of SGLT1 indicates that the prognosis of the patient is good or possibly good, and
   (b) a case that the patient is a non-diabetes mellitus patient and negative for expression of SGLT1 indicates that the prognosis of the patient is poor or possibly poor.
[18A] The method according to any one of [11A] to [17A], wherein the lung adenocarcinoma is early-stage cancer selected from the group consisting of Stage I cancer and Stage II cancer.
[19A] A pharmaceutical composition containing an insulin resistance improver, for use in treating a subject having diabetes mellitus and lung adenocarcinoma.
[20A] The pharmaceutical composition according to [19A], wherein the insulin resistance improver is one or more selected from the group consisting of biguanide drugs and thiazolidine drugs (a thiazolidinedione drug or glitazone) .
[21A] A pharmaceutical composition containing an SGLT2 inhibitor, for use in combination with an insulin resistance improver and in treating a subject having diabetes mellitus and lung adenocarcinoma.
[22A] A pharmaceutical composition containing an SGLT2 inhibitor, for use in treating a subject having diabetes mellitus and lung adenocarcinoma, wherein the subject has been treated with an insulin resistance improver or is planed to be treated therewith.
[23A] A pharmaceutical composition containing an insulin resistance improver, for use in combination with an SGLT2 inhibitor and in treating a subject having diabetes mellitus and lung adenocarcinoma.
[24A] A combination product including an SGLT2 inhibitor and an insulin resistance improver, for use in treating a subject having diabetes mellitus and lung adenocarcinoma.

### Brief Description of Drawings

[Figure 1] Figure 1 shows recurrence-free survival curves (DFI) for 102 lung adenocarcinoma cases. Stage I group is compared with an other-stage group.
[Figure 2] Figure 2 shows recurrence-free survival curves for 102 lung adenocarcinoma cases. Stage I and II group is compared with an other-stage group.
[Figure 3] Figure 3 shows survival curves for 102 lung adenocarcinoma cases. Stage I group is compared with an other-stage group.

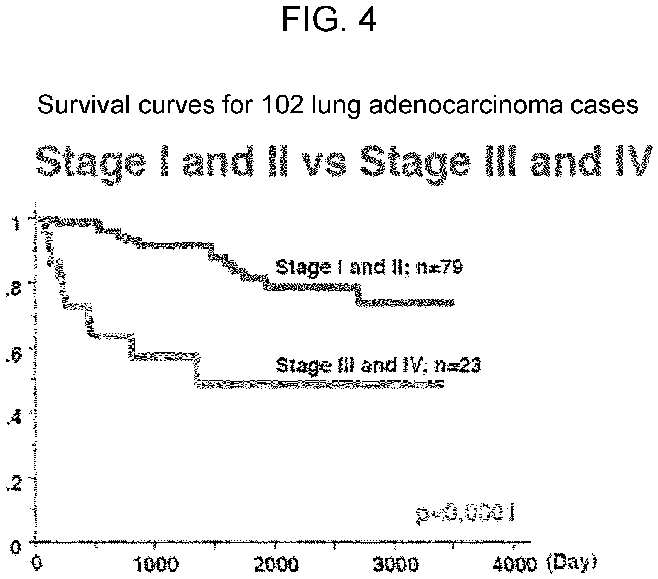

[Figure 4] Figure 4 shows survival curves for 102 lung adenocarcinoma cases. Stage I and II group is compared with an other-stage group.
[Figure 5] Figure 5 shows recurrence-free survival curves for 102 lung adenocarcinoma cases. Groups are compared on the basis of ratios of SGLT1-expressing cells to cancer cells. The threshold is 10%.
[Figure 6] Figure 6 shows recurrence-free survival curves for 102 lung adenocarcinoma cases. Groups are compared on the basis of ratios of SGLT2-expressing cells to cancer cells. The threshold is 10%.
[Figure 7] Figure 7 shows recurrence-free survival curves for 102 lung adenocarcinoma cases. Groups are compared on the basis of ratios of SGLT2-expressing cells to cancer cells. The threshold is 20%.
[Figure 8] Figure 8 shows survival curves for 102 lung adenocarcinoma cases. Groups are compared on the basis of ratios of SGLT1-expressing cells to cancer cells. The threshold is 10%.
[Figure 9] Figure 9 shows survival curves for 102 lung adenocarcinoma cases. Groups are compared on the basis of ratios of SGLT2-expressing cells to cancer cells. The threshold is 10%.
[Figure 10] Figure 10 shows survival curves for 102 lung adenocarcinoma cases. Groups are compared on the basis of ratios of SGLT2-expressing cells to cancer cells. The threshold is 20%.
[Figure 11] Figure 11 shows recurrence-free survival curves for Stage I lung adenocarcinoma. Groups are compared on the basis of ratios of SGLT1-expressing cells to cancer cells. The threshold is 10%.
[Figure 12] Figure 12 shows recurrence-free survival curves for Stage I lung adenocarcinoma. Groups are compared on the basis of ratios of SGLT2-expressing cells to cancer cells. The threshold is 10%.
[Figure 13] Figure 13 shows recurrence-free survival curves for Stage I lung adenocarcinoma. Groups are compared on the basis of ratios of SGLT2-expressing cells to cancer cells. The threshold is 20%.
[Figure 14] Figure 14 shows survival curves for Stage I lung adenocarcinoma. Groups are compared on the basis of ratios of SGLT1-expressing cells to cancer cells. The threshold is 10%.
[Figure 15] Figure 15 shows survival curves for Stage I lung adenocarcinoma. Groups are compared on the basis of ratios of SGLT2-expressing cells to cancer cells. The threshold is 10%.
[Figure 16] Figure 16 shows survival curves for Stage I lung adenocarcinoma. Groups are compared on the basis of ratios of SGLT2-expressing cells to cancer cells. The threshold is 20%.
[Figure 17] Figure 17 shows recurrence-free survival curves for Stage I and II lung adenocarcinoma. Groups are compared on the basis of ratios of SGLT1-expressing cells to cancer cells. The threshold is 10%.
[Figure 18] Figure 18 shows recurrence-free survival curves for Stage I and II lung adenocarcinoma. Groups are compared on the basis of ratios of SGLT2-expressing cells to cancer cells. The threshold is 10%.
[Figure 19] Figure 19 shows recurrence-free survival curves for Stage I and II lung adenocarcinoma. Groups are compared on the basis of ratios of SGLT2-expressing cells to cancer cells. The threshold is 20%.
[Figure 20] Figure 20 shows survival curves for Stage I and II lung adenocarcinoma. Groups are compared on the basis of ratios of SGLT1-expressing cells to cancer cells. The threshold is 10%.
[Figure 21] Figure 21 shows survival curves for Stage I and II lung adenocarcinoma. Groups are compared on the basis of ratios of SGLT2-expressing cells to cancer cells. The threshold is 10%.
[Figure 22] Figure 22 shows survival curves for Stage I and II lung adenocarcinoma. Groups are compared on the basis of ratios of SGLT2-expressing cells to cancer cells. The threshold is 20%.
[Figure 23] Figure 23 shows recurrence-free survival curves for 102 lung adenocarcinoma cases. A group aged 70 years or older is compared with a group aged younger than 70 years.
[Figure 24] Figure 24 shows recurrence-free survival curves for 102 lung adenocarcinoma cases. Four groups are compared, the groups resulting from classification in terms of whether a patient is aged 70 years or older or not and whether a patient is SGLT1-positive or not (threshold: 10%).
[Figure 25] Figure 25 shows recurrence-free survival curves for 102 lung adenocarcinoma cases. Four groups are compared, the groups resulting from classification in terms of whether a patient is aged 70 years or older or not and whether a patient is SGLT2-positive or not (threshold: 10%).
[Figure 26] Figure 26 shows survival curves for 102 lung adenocarcinoma cases. Four groups are compared, the groups resulting from classification in terms of whether a patient is aged 70 years or older or not and whether a patient is SGLT1-positive or not (threshold: 10%).
[Figure 27] Figure 27 shows recurrence-free survival curves for 102 lung adenocarcinoma cases. A group of diabetes mellitus patients is compared with a group of patients without diabetes mellitus.
[Figure 28] Figure 28 shows recurrence-free survival curves for 102 lung adenocarcinoma cases. Four groups are compared, the groups resulting from classification in terms of whether a patient is a diabetes mellitus patient or not and whether a patient is SGLT1-positive or not (threshold: 10%).
[Figure 29] Figure 29 shows recurrence-free survival curves for 102 lung adenocarcinoma cases. Four groups are compared, the groups resulting from classification in terms of whether a patient is a diabetes mellitus patient or not and whether a patient is SGLT2-positive or not (threshold: 10%).
[Figure 30] Figure 30 shows survival curves for 102 lung adenocarcinoma cases. A group of diabetes mellitus patients is compared with a group of patients without diabetes mellitus.
[Figure 31] Figure 31 shows survival curves for 102 lung adenocarcinoma cases. Four groups are compared, the groups resulting from classification in terms of whether a patient is a diabetes mellitus patient or not and whether a patient is SGLT1-positive or not (threshold: 10%) .
[Figure 32] Figure 32 shows recurrence-free survival curves for 102 lung adenocarcinoma cases. Four groups are compared, the groups resulting from classification in terms of whether a patient has a high CEA value (0.5 ng/mL or more) or not and whether a patient is SGLT1-positive or not (threshold: 10%).
[Figure 33] Figure 33 shows recurrence-free survival curves for 102 lung adenocarcinoma cases. Four groups are compared, the groups resulting from classification in terms of whether a patient has a high CEA value (0.5 ng/mL or more) or not and whether a patient is SGLT2-positive or not (threshold: 10%).
[Figure 34] Figure 34 shows recurrence-free survival curves for 102 lung adenocarcinoma cases. Four groups are compared, the groups resulting from classification in terms of whether a patient has a high CEA value (0.5 ng/mL or more) or not and whether a patient is SGLT2-positive or not (threshold: 20%).
[Figure 35] Figure 35 shows survival curves for 102 lung adenocarcinoma cases. Four groups are compared, the groups resulting from classification in terms of whether a patient has a high CEA value (0.5 ng/mL or more) or not and whether a patient is SGLT1-positive or not (threshold: 10%).
[Figure 36] Figure 36 shows survival curves for 102 lung adenocarcinoma cases. Four groups are compared, the groups resulting from classification in terms of whether a patient has a high CEA value (0.5 ng/mL or more) or not and whether a patient is SGLT2-positive or not (threshold: 10%).
[Figure 37] Figure 37 shows survival curves for 102 lung adenocarcinoma cases. Four groups are compared, the groups resulting from classification in terms of whether a patient has a high CEA value (0.5 ng/mL or more) or not and whether a patient is SGLT2-positive or not (threshold: 20%).
[Figure 38] Figure 38 shows recurrence-free survival curves for 102 lung adenocarcinoma cases. Four groups are compared, the groups resulting from classification in terms of whether a patient is SGLT1-positive or not (threshold: 10%) and whether a patient is SGLT2-positive or not (threshold: 10%).
[Figure 39] Figure 39 shows recurrence-free survival curves for 102 lung adenocarcinoma cases. Four groups are compared, the groups resulting from classification in terms of whether a patient is SGLT1-positive or not (threshold: 10%) and whether a patient is SGLT2-positive or not (threshold: 20%).
[Figure 40] Figure 40 shows survival curves for 102 lung adenocarcinoma cases. Groups are compared on the basis of ratios of SGLT1-expressing cells to cancer cells. The threshold is 10%.
[Figure 41] Figure 41 shows recurrence-free survival curves for 102 lung adenocarcinoma cases. Groups are compared on the basis of ratios of SGLT2-expressing cells to cancer cells. The threshold is 10%.
[Figure 42] Figure 42 shows recurrence-free survival curves for 102 lung adenocarcinoma cases. Groups are compared on the basis of ratios of SGLT2-expressing cells to cancer cells. The threshold is 20%.
[Figure 43] Figure 43 shows recurrence-free survival curves for 102 lung adenocarcinoma cases. A group of diabetes mellitus patients is compared with a group of patients without diabetes mellitus.
[Figure 44] Figure 44 shows recurrence-free survival curves for 102 lung adenocarcinoma cases. A group with EGFR mutation is compared with a group without EGFR mutation.
[Figure 45] Figure 45 shows recurrence-free survival curves for 102 lung adenocarcinoma cases. A group of diabetes mellitus patients is compared with a group of patients without diabetes mellitus.
[Figure 46] Figure 46 shows survival curves for 102 lung adenocarcinoma cases. A group of diabetes mellitus patients is compared with a group of patients without diabetes mellitus.
[Figure 47] Figure 47 shows recurrence-free survival curves for Stage I and II lung adenocarcinoma. A group of diabetes mellitus patients is compared with a group of patients without diabetes mellitus.
[Figure 48] Figure 48 shows survival curves for Stage I and II lung adenocarcinoma. A group of diabetes mellitus patients is compared with a group of patients without diabetes mellitus.
[Figure 49] Figure 49 shows the influence of being affected by diabetes mellitus on the survival rate of lung cancer patients.
[Figure 50] Figure 50 shows the influence of being affected by diabetes mellitus on the recurrence-free survival time of lung cancer patients.
[Figure 51] Figure 51 shows the influence of being affected by diabetes mellitus on the survival rate of small cell lung cancer patients.
[Figure 52] Figure 52 shows the influence of being affected by diabetes mellitus on the survival rate of small cell lung cancer patients after surgical therapy.
[Figure 53] Figure 53 shows the influence of diabetes mellitus treatment on the recurrence-free survival time of lung adenocarcinoma patients.

### Description of Embodiments

### <Definition>

Herein, "subjects" can be each a mammal, and are preferably each a human. The subjects can be each a subject affected by tumor or cancer, or having a risk thereof. The subjects can include subjects aged 70 years or older and subjects aged younger than 70 years. The subjects can include smokers (e.g., BI index: 700 or higher) and non-smokers.

Herein, "cancer" is a disease characterized by uncontrolled cell proliferation. Cancer cells include ones that locally propagate and ones that systemically propagate through the blood stream or the lymphatic system. Non-limiting examples of cancer include solid tumors. Non-limiting examples of cancer include hematopoietic tumors. Examples of solid tumors include lung cancer. Herein, "cancer tissue" is tissue containing cancer. Cancer tissue can be obtained through biopsy and cancer resection. Herein, the term "prognosis" can mean a cancer recurrence rate (or period until recurrence) and survival rate. Recurrences include ones in primary lesions and ones after metastasis. Recurrence rates can be ones after a specific period of treatment. The specific period can be, for example, 1 year, 2 years, 3 years, 4 years, 5 years, 6 years, 7 years, 8 years, 9 years, 10 years, 11 years, 12 years, 13 years, 14 years, or 15 years, or a period equal to or longer than any of them.

Herein, "lung adenocarcinoma" is one of lung cancers. Lung cancers are roughly classified into small cell lung cancers and non-small-cell lung cancers, and non-small-cell lung cancers are roughly classified into three cancers including lung adenocarcinoma. Non-small-cell lung cancers account for approximately 80% of lung cancers, and lung adenocarcinoma accounts for approximately 60% of non-small-cell lung cancers.

Herein, the term "gene expression" or expressions similar thereto mean both expression of messenger RNA (mRNA) and expression of protein, from a gene present on the genome of a cell. mRNA can be detected by a method well known to those skilled in the art through the process that total RNA is extracted from cells, the first strand of cDNA is synthesized, for example, with use of a poly A sequence uniquely possessed by mRNA, and amplification (e.g., polymerase chain reaction) is subsequently performed. Protein can be detected by using an antibody (e.g., a polyclonal antibody or a monoclonal antibody) that specifically binds to the protein.

Herein, the term "positive" in the context of gene expression in cells means that mRNA or protein is detected at a detectable level, unless otherwise stated. The term "negative" means that mRNA or protein is not detected at a detectable level, unless otherwise stated. Herein, a threshold is set to clarify the classification of "positive" and "negative" in the context of gene expression in tissue, and cases with an expression level equal to or higher than the threshold are regarded as being "positive", and cases with an expression level lower than the threshold are regarded as being "negative". Thus, being "positive" for gene expression at the cell level and being "positive" for gene expression at the tissue level are herein different with respect to determination criteria.

Herein, an expression level refers to the amount of mRNA or protein expressed in tissue or a specific cell population. The expression level of mRNA can be quantified by various methods well known to those skilled in the art for quantifying mRNA such as quantitative PCR. Proteins can be quantified by a method well known to those skilled in the art such as immunohistochemical staining (IHC). In IHC, an antibody binds to a protein in a tissue section, and the antibody can be allowed to color by the action of a dye. The intensity of coloring corresponds to the expression level of the protein. Thus, the expression level of a protein can be quantified via the intensity of coloring. In IHC, the expression level of a protein can also be quantified on the basis not of the intensity of coloring, but of the number or ratio of cells with expression. For the expression level of a protein, quantification based on the intensity of coloring or one based on the number or ratio of cells with expression is selected according to the situation and the purpose.

Herein, "SGLT" is a sodium-dependent glucose transporter, and serves as a sodium-glucose coupled transporter. Examples of SGLT include SGLT1, SGLT2, and SGLT3. SGLT1 is expressed in renal tubules, intestines, myocardia, and others, and SGLT2 is limitedly expressed in proximal renal tubules in kidneys. Proximal renal tubules take necessary substances out of the blood and incorporate them into the body, and excrete unnecessary substances as urine. Proximal renal tubules resorb glucose once excreted and incorporate it into the blood. It is believed that 90% of the glucose to be resorbed is due to SGLT2 and the rest 10% is due to SGLT1.

Human SGLT1 is known to be present on a region of human chromosome 22 (22q12.3; NC_000022.11; 32043050..32113029), the region located between RPS17P16 and AP1B1P1. Human SGLT1 protein can have, for example, an amino acid sequence registered under GenBank Accession No. AAB59448.1, or an amino acid sequence corresponding thereto. The human SGLT1 protein may have a mutation selected from the group consisting of insertion, deletion, addition, and substitution of one to several amino acids. The human SGLT1 protein may have an amino acid sequence identity of 80% or more, 85% or more, 90% or more, or 95% or more with the amino acid sequence registered under GenBank Accession No. AAB59448.1. The human SGLT1 protein can retain the functions of a sodium-dependent glucose transporter.

Human SGLT2 is known to be present on a region of human chromosome 16 (16p11.2; NC_000016.10; specifically, 31482535..31490769), the region located between TGFB1T1 and RUSF1. Human SGLT2 protein is encoded by the chromosomal region, and can have, for example, an amino acid sequence registered as NCBI Reference Sequence: NP_003032.1, or an amino acid sequence corresponding thereto. The human SGLT2 protein may have a mutation selected from the group consisting of insertion, deletion, addition, and substitution of one to several amino acids. The human SGLT2 protein may have an amino acid sequence identity of 80% or more, 85% or more, 90% or more, or 95% or more with the amino acid sequence registered under GenBank Accession No. AAB59448.1. The human SGLT2 protein can retain the functions of a sodium-dependent glucose transporter.

Herein, the term "diabetes mellitus" means a disease with a sustained high blood glucose level. Specifically, diagnosis of diabetes mellitus can be based on the following criteria {e.g., fasting blood glucose ≥ 126 mg/dL, HbAlc ≥ 6.5%, 2-hour value in oral glucose tolerance test (75 g OGTT): 200 mg/dL or more}. Diabetes mellitus is roughly classified into type 1 diabetes mellitus and type 2 diabetes mellitus. Type 1 diabetes mellitus is a disease induced by insufficient secretion of insulin, which controls blood glucose, due to destruction of β cells, which are insulin-producing cells in the pancreas. Type 2 diabetes mellitus is a disease induced by decrease in insulin secretion from β cells or lowering of the responsiveness of cells to insulin in glucose uptake, or lowering of the ability of glucose uptake into muscles and fat tissues. Various medicaments have been developed for diabetes mellitus, and SGLT2 inhibitors (e.g., ipragliflozin, dapagliflozin, luseogliflozin, tofogliflozin, canagliflozin, and empagliflozin), the SGLT2 being responsible for resorption of glucose into the blood in proximal renal tubules, are expected to be promising in diabetes mellitus treatment.

### <Method of Present Invention for Predicting Prognosis (SGLT1)>

(A) The present invention provides
   a method for predicting a prognosis of a cancer patient, the method including:
   a detection step of detecting expression of SGLT1 in a cancer tissue obtained from a cancer patient.

In (A), if the cancer tissue is negative for SGLT1, the prognosis can be predicted to be poor or possibly poor (or a poor prognosis or the possibility of a poor prognosis is indicated). In (A), if the cancer tissue is positive for SGLT1, the prognosis can be predicted to be good or possibly good (or a good prognosis or the possibility of a good prognosis is indicated). In (A), if the cancer tissue is negative for SGLT1, the prognosis can be predicted to be poor or possibly poor (or a poor prognosis or the possibility of a poor prognosis is indicated), and if the cancer tissue is positive for SGLT1, the prognosis can be predicted to be good or possibly good (or a good prognosis or the possibility of a good prognosis is indicated).

In an embodiment, whether the cancer tissue is positive or negative for SGLT1 can be determined on the basis of the amount of SGLT1 in the tissue (or a section or cell homogenate thereof). In an embodiment, the detection can be performed by quantifying mRNA encoding SGLT1. The quantification can be performed by using a quantification method well known to those skilled in the art such as a method with quantitative PCR, a nextgeneration sequencer, or a microarray. In an embodiment, the detection can be performed by using an antibody that binds to SGLT1 (an anti-SGLT1 antibody). The tissue (or a section or cell homogenate thereof) is brought into contact with an antibody that binds to SGLT1 under conditions that allow the formation of a complex of SGLT1 protein and the antibody, and the formation of the complex allows SGLT1 protein to be detected. The detection can be performed by using a method well known to those skilled in the art such as a Western blotting method and a protein array method. On the basis of the detection level, the cancer tissue can be determined to be SGLT1-positive or -negative. In a preferred embodiment, immunohistochemical staining can confirm that the cancer tissue is positive or negative for SGLT1.

A first predetermined value can be equal to or lower than the maximum, third quartile, mean, or median of the expression levels of SGLT1 in a good-prognosis cancer patient group. The first predetermined value can be equal to or higher than the minimum, first quartile, mean, or median of the expression levels of SGLT1 in a poor-prognosis cancer patient group. The first predetermined value can be equal to or lower than the maximum, third quartile, mean, or median of the expression levels of SGLT1 in a good-prognosis cancer patient group, and can be equal to or higher than the minimum, first quartile, mean, or median of the expression levels of SGLT1 in a poor-prognosis cancer patient group. Here, whether the prognosis is good or poor can be determined by a physician. For example, whether the prognosis is good or poor can be determined in view of length of recurrence-free survival time or length of survival time. For example, a patient whose recurrence-free survival time after cancer resection is 1,000 hours or longer, 1,100 hours or longer, 1,200 hours or longer, 1,300 hours or longer, 1,400 hours or longer, 1,500 hours or longer, 1,600 hours or longer, 1,700 hours or longer, 1,800 hours or longer, 1,900 hours or longer, 2,000 hours or longer, 2,100 hours or longer, 2,200 hours or longer, 2,300 hours or longer, 2,400 hours or longer, 2,500 hours or longer, 2,600 hours or longer, 2,700 hours or longer, 2,800 hours or longer, 2,900 hours or longer, or 3,000 hours or longer can be determined as a patient having a good prognosis, and a patient whose recurrence-free survival time after cancer resection is shorter than the period (a patient who has been determined not to have a good prognosis) can be determined as a patient having a poor prognosis. For example, a patient whose survival time after cancer resection is 1,000 hours or longer, 1,100 hours or longer, 1,200 hours or longer, 1,300 hours or longer, 1,400 hours or longer, 1,500 hours or longer, 1,600 hours or longer, 1,700 hours or longer, 1,800 hours or longer, 1,900 hours or longer, 2,000 hours or longer, 2,100 hours or longer, 2,200 hours or longer, 2,300 hours or longer, 2,400 hours or longer, 2,500 hours or longer, 2,600 hours or longer, 2,700 hours or longer, 2,800 hours or longer, 2,900 hours or longer, or 3,000 hours or longer can be determined as a patient having a good prognosis, and a patient whose survival time after cancer resection is shorter than the period (a patient who has been determined not to have a good prognosis) can be determined as a patient having a poor prognosis.

In a preferred embodiment, whether the cancer tissue is positive or negative for SGLT1 can be determined on the basis of the ratio of SGLT1-positive cells to cancer cells in the cancer tissue. For example, if the ratio of SGLT1-positive cells is equal to or higher than the first predetermined value, the prognosis of the cancer patient from which the cancer tissue is derived can be predicted to be poor or possibly poor (or a poor prognosis or the possibility of a poor prognosis is indicated). For example, if the ratio of SGLT1-positive cells is lower than the first predetermined value, the prognosis can be predicted to be good or possibly good (or a good prognosis or the possibility of a good prognosis is indicated). If the ratio of SGLT1-positive cells is equal to or higher than the first predetermined value, the prognosis of the cancer patient from which the cancer tissue is derived can be predicted to be poor or possibly poor (or a poor prognosis or the possibility of a poor prognosis is indicated), and if the ratio of SGLT1-positive cells is equal to or higher than the first predetermined value, the prognosis of the cancer patient from which the cancer tissue is derived can be predicted to be poor or possibly poor (or a poor prognosis or the possibility of a poor prognosis is indicated).

The first predetermined value is a threshold (or a cutoff value) for the ratio of SGLT1-positive cells to cancer cells. In an embodiment, the first predetermined value can be a value of 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, or 30% or more. The first predetermined value can be, for example, a value of 5 to 30%, 5 to 25%, or 10 to 20%.

In a preferred embodiment, the cancer patient is a patient after cancer resection.

In a preferred embodiment, the cancer is lung cancer, and more preferably lung adenocarcinoma.

In a preferred embodiment, the cancer patient is a patient after cancer resection, and the cancer is lung adenocarcinoma.

In a preferred embodiment, the expression of SGLT1 is expression of SGLT1 protein. In a preferred embodiment, whether the cancer tissue obtained from the cancer patient is positive for SGLT1 is determined on the basis of the ratio of SGLT1-positive cells therein. In a preferred embodiment, if the ratio of SGLT1-positive cells in the cancer tissue obtained from the cancer patient is equal to or higher than the first predetermined value, the cancer tissue is determined to be SGLT1-positive. In a preferred embodiment, if the ratio of SGLT1-positive cells in the cancer tissue obtained from the cancer patient is lower than the first predetermined value, the cancer tissue is determined to be SGLT1-negative.

In a preferred embodiment, the cancer is lung adenocarcinoma, and if the ratio of SGLT1-positive cells in the cancer tissue obtained from the cancer patient is equal to or higher than the first predetermined value, the cancer tissue is determined to be SGLT1-positive.

### <Method of Present Invention for Predicting Prognosis (SGLT2)>

(B) The present invention provides, in another aspect,
a method for predicting a prognosis of a cancer patient, the method including:
a detection step of detecting expression of SGLT2 in a cancer tissue obtained from a cancer patient.

In (B), if the cancer tissue is negative for SGLT2, the prognosis can be predicted to be poor or possibly poor (or a poor prognosis or the possibility of a poor prognosis is indicated). In (B), if the cancer tissue is positive for SGLT2, the prognosis can be predicted to be good or possibly good (or a good prognosis or the possibility of a good prognosis is indicated). In (B), if the cancer tissue is negative for SGLT2, the prognosis can be predicted to be poor or possibly poor (or a poor prognosis or the possibility of a poor prognosis is indicated), and if the cancer tissue is positive for SGLT2, the prognosis can be predicted to be good or possibly good (or a good prognosis or the possibility of a good prognosis is indicated).

In an embodiment, whether the cancer tissue is positive or negative for SGLT2 can be determined on the basis of the amount of SGLT2 in the tissue (or a section or cell homogenate thereof). In an embodiment, the detection can be performed by quantifying mRNA encoding SGLT2. The quantification can be performed by using a quantification method well known to those skilled in the art such as a method with quantitative PCR, a nextgeneration sequencer, or a microarray. In an embodiment, the detection can be performed by using an antibody that binds to SGLT2 (an anti-SGLT2 antibody). The tissue (or a section or cell homogenate thereof) is brought into contact with an antibody that binds to SGLT2 under conditions that allow the formation of a complex of SGLT2 protein and the antibody, and the formation of the complex allows SGLT2 protein to be detected. The detection can be performed by using a method well known to those skilled in the art such as a Western blotting method and a protein array method. On the basis of the detection level, the cancer tissue can be determined to be SGLT2-positive or -negative. In a preferred embodiment, immunohistochemical staining can confirm that the cancer tissue is positive or negative for SGLT2.

A second predetermined value can be equal to or lower than the maximum, third quartile, mean, or median of the expression levels of SGLT2 in a good-prognosis cancer patient group. The second predetermined value can be equal to or higher than the minimum, second quartile, mean, or median of the expression levels of SGLT2 in a poor-prognosis cancer patient group. The second predetermined value can be equal to or lower than the maximum, third quartile, mean, or median of the expression levels of SGLT2 in a good-prognosis cancer patient group, and can be equal to or higher than the minimum, second quartile, mean, or median of the expression levels of SGLT2 in a poor-prognosis cancer patient group. Here, whether the prognosis is good or poor can be determined by a physician. For example, whether the prognosis is good or poor can be determined in view of length of recurrence-free survival time or length of survival time. For example, a patient whose recurrence-free survival time after cancer resection is 1,000 hours or longer, 1,100 hours or longer, 1,200 hours or longer, 1,300 hours or longer, 1,400 hours or longer, 1,500 hours or longer, 1,600 hours or longer, 1,700 hours or longer, 1,800 hours or longer, 1,900 hours or longer, 2,000 hours or longer, 2,100 hours or longer, 2,200 hours or longer, 2,300 hours or longer, 2,400 hours or longer, 2,500 hours or longer, 2,600 hours or longer, 2,700 hours or longer, 2,800 hours or longer, 2,900 hours or longer, or 3,000 hours or longer can be determined as a patient having a good prognosis, and a patient whose recurrence-free survival time after cancer resection is shorter than the period (a patient who has been determined not to have a good prognosis) can be determined as a patient having a poor prognosis. For example, a patient whose survival time after cancer resection is 1,000 hours or longer, 1,100 hours or longer, 1,200 hours or longer, 1,300 hours or longer, 1,400 hours or longer, 1,500 hours or longer, 1,600 hours or longer, 1,700 hours or longer, 1,800 hours or longer, 1,900 hours or longer, 2,000 hours or longer, 2,100 hours or longer, 2,200 hours or longer, 2,300 hours or longer, 2,400 hours or longer, 2,500 hours or longer, 2,600 hours or longer, 2,700 hours or longer, 2,800 hours or longer, 2,900 hours or longer, or 3,000 hours or longer can be determined as a patient having a good prognosis, and a patient whose survival time after cancer resection is shorter than the period (a patient who has been determined not to have a good prognosis) can be determined as a patient having a poor prognosis.

In a preferred embodiment, whether the cancer tissue is positive or negative for SGLT2 can be determined on the basis of the ratio of SGLT2-positive cells to cancer cells in the cancer tissue. For example, if the ratio of SGLT2-positive cells is equal to or higher than the second predetermined value, the prognosis of the cancer patient from which the cancer tissue is derived can be predicted to be poor or possibly poor (or a poor prognosis or the possibility of a poor prognosis is indicated). For example, if the ratio of SGLT2-positive cells is lower than the second predetermined value, the prognosis can be predicted to be good or possibly good (or a good prognosis or the possibility of a good prognosis is indicated). If the ratio of SGLT2-positive cells is equal to or higher than the second predetermined value, the prognosis of the cancer patient from which the cancer tissue is derived can be predicted to be poor or possibly poor (or a poor prognosis or the possibility of a poor prognosis is indicated), and if the ratio of SGLT2-positive cells is equal to or higher than the second predetermined value, the prognosis of the cancer patient from which the cancer tissue is derived can be predicted to be poor or possibly poor (or a poor prognosis or the possibility of a poor prognosis is indicated).

The second predetermined value is a threshold (or a cutoff value) for the ratio of SGLT2-positive cells to cancer cells. In an embodiment, the second predetermined value can be a value of 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, or 30% or more. The second predetermined value can be, for example, a value of 5 to 30%, 5 to 25%, or 10 to 20%.

In a preferred embodiment, the cancer patient is a patient after cancer resection.

In a preferred embodiment, the cancer is lung cancer, and more preferably lung adenocarcinoma.

In a preferred embodiment, the cancer patient is a patient after cancer resection, and the cancer is lung adenocarcinoma.

In a preferred embodiment, the expression of SGLT2 is expression of SGLT2 protein. In a preferred embodiment, whether the cancer tissue obtained from the cancer patient is positive for SGLT2 is determined on the basis of the ratio of SGLT2-positive cells therein. In a preferred embodiment, if the ratio of SGLT2-positive cells in the cancer tissue obtained from the cancer patient is equal to or higher than the second predetermined value, the cancer tissue is determined to be SGLT2-positive. In a preferred embodiment, if the ratio of SGLT2-positive cells in the cancer tissue obtained from the cancer patient is lower than the second predetermined value, the cancer tissue is determined to be SGLT2-negative.

In a preferred embodiment, the cancer is lung adenocarcinoma, and if the ratio of SGLT2-positive cells in the cancer tissue obtained from the cancer patient is equal to or higher than the second predetermined value, the cancer tissue is determined to be SGLT2-positive.

### <Method of Present Invention for Predicting Prognosis (SGLT1 and SGLT2)>

According to the present invention, a prognosis of a cancer patient can be predicted with a combination of (A) and (B). For example, the method of the present invention for predicting a prognosis can be a method including:
a detection step of detecting expression of SGLT1 in a cancer tissue obtained from a cancer patient; and
a detection step of detecting expression of SGLT2 in the cancer tissue obtained from the cancer patient.

In the method of the present invention, if the cancer tissue is negative for SGLT1 and/or positive for SGLT2, the prognosis of the cancer patient can be predicted to be poor (or a poor prognosis or the possibility of a poor prognosis is indicated). In the method of the present invention, if the cancer tissue is positive for SGLT1 and/or negative for SGLT2, the prognosis of the cancer patient can be predicted to be good (or a good prognosis or the possibility of a good prognosis is indicated). In the method of the present invention, for example, if the cancer tissue is negative for SGLT1 and positive for SGLT2, the prognosis of the cancer patient can be predicted to be poor (or a poor prognosis or the possibility of a poor prognosis is indicated), and if the cancer tissue is positive for SGLT1 and negative for SGLT2, the prognosis of the cancer patient can be predicted to be good (or a good prognosis or the possibility of a good prognosis is indicated).

(A) and (B) in the present invention are as described above, and hence redundant description will be omitted; see the above description.

In a preferred embodiment, the cancer patient is a patient after cancer resection.

In a preferred embodiment, the cancer is lung cancer, and more preferably lung adenocarcinoma.

In a preferred embodiment, the cancer patient is a patient after cancer resection, and the cancer is lung adenocarcinoma.

In a preferred embodiment, the expression of SGLT1 is expression of SGLT1 protein. In a preferred embodiment, whether the cancer tissue obtained from the cancer patient is positive for SGLT1 is determined on the basis of the ratio of SGLT1-positive cells therein. In a preferred embodiment, if the ratio of SGLT1-positive cells in the cancer tissue obtained from the cancer patient is equal to or higher than the first predetermined value, the cancer tissue is determined to be SGLT1-positive. In a preferred embodiment, if the ratio of SGLT1-positive cells in the cancer tissue obtained from the cancer patient is lower than the first predetermined value, the cancer tissue is determined to be SGLT1-negative. The first predetermined value can be, for example, a value of 5 to 30%, 5 to 25%, or 10 to 20%.

In a preferred embodiment, the expression of SGLT2 is expression of SGLT2 protein. In a preferred embodiment, whether the cancer tissue obtained from the cancer patient is positive for SGLT2 is determined on the basis of the ratio of SGLT2-positive cells therein. In a preferred embodiment, if the ratio of SGLT2-positive cells in the cancer tissue obtained from the cancer patient is equal to or higher than the second predetermined value, the cancer tissue is determined to be SGLT2-positive. In a preferred embodiment, if the ratio of SGLT2-positive cells in the cancer tissue obtained from the cancer patient is lower than the second predetermined value, the cancer tissue is determined to be SGLT2-negative. The second predetermined value can be, for example, a value of 5 to 30%, 5 to 25%, or 10 to 20%.

<Further Aspect of Present Invention>

The present invention provides a method for predicting a prognosis of a cancer patient, the method including:
(2) predicting that the prognosis is good if the patient is a diabetes mellitus patient, and predicting that the prognosis is poor if the patient is a non-diabetes mellitus patient.

Whether the cancer patient has diabetes mellitus can be determined through diagnosis by a physician. In an embodiment, the cancer patient can be a cancer patient having been subjected to SGLT2 inhibitor therapy.

In addition, the present invention provides a method for predicting a prognosis of a cancer patient, the method including:
(3) predicting that the prognosis is good if the patient has a mutation in epidermal growth factor receptor (EGFR), and predicting that the prognosis is poor if the patient has no mutation in EGFR.

The mutation in EGFR can be a driver mutation in EGFR. A driver mutation in EGFR is a mutation that enhances the activity of EGFR and causes cancer. EGFR refers to epidermal growth factor receptor, and provides a cell with a proliferative signal on binding to epidermal growth factor (EGF) as a ligand. The cell provided with the proliferative signal proliferates. The driver mutation in EGFR switches on or enhances the proliferative signal. Examples of such driver mutations in EGFR include any mutation selected from the group consisting of G719X, deletion in exon 19 (del 19), S768I, insertion in exon 20 (ins 20), T790M, L858R, and L861Q, wherein X represents any amino acid other than G. These driver mutations are found in cancer, and enhance the activity of EGFR or constitutively activate EGFR. G719X, deletion in exon 19 (del 19), and L858R contribute to high response rates for EGFR-tyrosine kinase inhibitors (EGFR-TKI), and T790M and insertion in exon 20 relate to treatment resistance to EGFR-TKI. Examples of deletion in exon 19 (del 19) include, but are not limited to, delE746 _A750. Examples of insertion in exon 20 (ins 20) include, but are not limited to, V769_D770insASV, D770_N771insSVD, and H773_V774insH. Examples of G719X include, but are not limited to, G719A, G719S, and G719C.

Examples of cancer patients having a driver mutation in EGFR include a cancer patient having been subjected to EGFR-tyrosine kinase inhibitor (EGFR-TKI) therapy. Examples of EGFR-tyrosine kinase inhibitors (EGFR-TKI) include first-generation EGFR-TKIs such as gefitinib and erlotinib; second-generation EGFR-TKIs such as afatinib and dacomitinib; and third-generation EGFR-TKIs such as osimertinib.

The method of the present invention for predicting a cancer prognosis can be a combination of two, three, or four selected from the group consisting of (A), (B), (2), and (3). For example, the method of the present invention for predicting a cancer prognosis can include (A) and (2). For example, the method of the present invention for predicting a cancer prognosis can include (A) and (3). For example, the method of the present invention for predicting a cancer prognosis can include (A), (2), and (3). For example, the method of the present invention for predicting a cancer prognosis can include (B) and (2). For example, the method of the present invention for predicting a cancer prognosis can include (B) and (3). For example, the method of the present invention for predicting a cancer prognosis can include (B), (2), and (3). For example, the method of the present invention for predicting a cancer prognosis can include (A), (B), and (2). For example, the method of the present invention for predicting a cancer prognosis can include (A), (B), and (3). For example, the method of the present invention for predicting a cancer prognosis can include (A), (B), (2), and (3).

In each of (A), (B), (2), and (3), whether the prognosis is good or poor can be predicted. If all the predictions agree with the prediction that the prognosis is good, the prognosis of the patient from which the cancer tissue is derived is predicted to be good (or a good prognosis or the possibility of a good prognosis is indicated). If all the predictions agree with the prediction that the prognosis is poor, the prognosis of the patient from which the cancer tissue is derived is predicted to be poor (or a poor prognosis or the possibility of a poor prognosis is indicated). If no complete agreement is found among the predictions, the prognosis can be remained unpredicted, or predicted to be good or poor according to the purpose. For the purpose of treatment, for example, the prognosis can be predicted to be poor for considering selection of more efficient treatment. Even if no complete agreement is found among the predictions, the prognosis of the patient from which the cancer tissue is derived can be predicted to be good (or a good prognosis or the possibility of a good prognosis is indicated) if the number of predictions that the prognosis can be good is larger than the number of predictions that the prognosis is poor. Even if no complete agreement is found among the predictions, the prognosis of the patient from which the cancer tissue is derived can be predicted to be poor (or a poor prognosis or the possibility of a poor prognosis can be indicated) if the number of predictions that the prognosis is poor is larger than the number of predictions that the prognosis is good. If the prognosis is predicted neither to be good nor to be poor, for example, a prediction result that the prognosis can be said neither to be good nor to be poor can be returned.

The method of the present invention for predicting a prognosis can be an industrially applicable invention. The method of the present invention for predicting a prognosis can be an in vitro method. The method of the present invention for predicting a prognosis can be a method for predicting a prognosis, an auxiliary method for predicting a prognosis, or a method for acquiring basic information for predicting a prognosis. The method of the present invention for predicting a prognosis can be free of a step of diagnosis by a physician. After performing the method of the present invention for predicting a prognosis, a physician can diagnose the prognosis. The method of the present invention for predicting a prognosis can be free of a step of diagnosing an animal (in particular, a human).

### <Pharmaceutical Composition of Present Invention>

The present invention provides a pharmaceutical composition containing an SGLT2 inhibitor for use in treating cancer in an SGLT2-positive cancer patient. The cancer can be lung cancer, and preferably be lung adenocarcinoma. The cancer patient can be a cancer patient predicted to have a good prognosis by the above method of the present invention for predicting a prognosis. The cancer patient can be a cancer patient predicted to have a poor prognosis by the above method of the present invention for predicting a prognosis. The cancer patient can be a cancer patient predicted neither to have a good prognosis nor to have a poor prognosis by the above method of the present invention for predicting a prognosis. In an embodiment, the cancer patient can be a patient having unresectable or recurrent cancer. In an embodiment, the cancer patient can be a patient after cancer resection. In an embodiment, the cancer patient is affected by diabetes mellitus and cancer. In an embodiment, the cancer patient has a driver mutation in EGFR. In an embodiment, the cancer patient has a driver mutation in EGFR, and is affected by diabetes mellitus and cancer. In an embodiment, the cancer patient has no driver mutation in EGFR, and is affected by diabetes mellitus and cancer. In a preferred embodiment of the present invention, the pharmaceutical composition of the present invention can be used in combination with an insulin resistance improver for a lung adenocarcinoma patient having diabetes mellitus. In a preferred embodiment of the present invention, the pharmaceutical composition of the present invention can be administrated to a lung adenocarcinoma patient having diabetes mellitus, the patient having been treated with an insulin resistance improver or scheduled to be treated with an insulin resistance improver.

According to the present invention, the SGLT2-positive cancer patient can be a patient after cancer resection. According to the present invention, the SGLT2-positive cancer patient is a cancer patient such that the ratio of SGLT2-positive cells to cancer cells in immunohistochemical staining of the cancer tissue is equal to or higher than the second predetermined value. The second predetermined value can be, for example, a value of 5 to 30%, 5 to 25%, or 10 to 20%.

According to the present invention, the SGLT2-positive cancer patient can be SGLT1-positive. According to the present invention, the SGLT2-positive cancer patient can be SGLT1-negative. According to the present invention, the SGLT2-positive cancer patient is a cancer patient such that the ratio of SGLT2-positive cells to cancer cells in immunohistochemical staining of the cancer tissue is equal to or higher than the first predetermined value. The first predetermined value can be, for example, a value of 5 to 30%, 5 to 25%, or 10 to 20%.

The present invention provides an SGLT2 inhibitor for use in treating cancer. The present invention provides use of an SGLT2 inhibitor in manufacture of a medicament for use in treating cancer. The present invention provides a method for treating cancer in a subject having cancer, the method including administrating a therapeutically effective amount of an SGLT2 inhibitor to the patient. The patient can be a subject predicted to have a good prognosis by the method of the present invention for predicting a prognosis. The patient can be a subject predicted to have a poor prognosis by the method of the present invention for predicting a prognosis. The patient can be a subject predicted neither to have a good prognosis nor to have a poor prognosis by the method of the present invention for predicting a prognosis. According to the present invention, for example, administration of an SGLT2 inhibitor to a patient having a good prognosis can allow the patient to avoid the affliction due to unnecessary treatment. According to the present invention, for example, even in the case that an SGLT2 inhibitor causes a severe side effect, treatment by administrating the SGLT2 inhibitor can be applied only to subjects tolerant to the side effect among patients having a poor prognosis. In another case, an increased dose of an SGLT2 inhibitor can be administrated to a patient having a poor prognosis. The present invention provides use of an SGLT2 inhibitor in manufacture of a medicament for use in treating cancer. Each SGLT2 inhibitor can be any SGLT2 inhibitor shown above as examples or a pharmaceutical product containing this SGLT2 inhibitor.

An SGLT2 inhibitor can be contained in a pharmaceutical composition, and the pharmaceutical composition can contain an SGLT2 inhibitor and a pharmaceutically acceptable carrier, diluent, and/or excipient. The pharmaceutical composition can be formulated for oral administration or parenteral administration.

The present invention provides a pharmaceutical composition containing an insulin resistance improver for use in treating a lung adenocarcinoma patient having diabetes mellitus. In addition, the present invention provides use of an insulin resistance improver in manufacture of a medicament for use in treating a lung adenocarcinoma patient having diabetes mellitus. Moreover, the present invention provides a method for treating a lung adenocarcinoma patient having diabetes mellitus, the method including administrating a therapeutically effective amount of an insulin resistance improver to the patient. In an embodiment, the therapeutically effective amount of an insulin resistance improver improves the prognosis of the patient (e.g., the survival rate and the recurrence-free survival time). Examples of the insulin resistance improver include a biguanide drug and a PPARy agonist (e.g., a thiazolidine drug). Examples of the biguanide drug include metformin, buformin, and phenformin. Examples of the thiazolidine drug include isaglitazone, (S)-((3,4-dihydro-2-(phenylmethyl)-2H-1-benzopyran-6-yl)methyl-thiazolidine-2,4-dione (englitazone), 5-{[4-(3-(5-methyl-2-phenyl-4-oxazolyl)-1-oxopropyl)-phenyl]-methyl}-thiazolidine-2,4-dione (darglitazone), 5-{[4-(1-methylcyclohexyl)methoxy]-phenyl}methyl)-thiazolidine-2,4-dione (ciglitazone), 5-{[4-(2-(1-indolyl)ethoxy)phenyl]methyl}-thiazolidine-2,4-dione (DRF2189), 5-{4-[2-(5-methyl-2-phenyl-4-oxazolyl)-ethoxy]benzyl}-thiazolidine-2,4-dione (BM-13.1246), 5-(2-naphthylsulfonyl)-thiazolidine-2,4-dione (AY-31637), bis{4-[(2,4-dioxo-5-thiazolidinyl)methyl]phenyl}methane (YM268), 5-{4-[2-(5-methyl-2-phenyl-4-oxazolyl)-2-hydroxyethoxy]benzyl}-thiazolidine-2,4-dione (AD-5075), 5-[4-(1-phenyl-1-cyclopropanecarbonylamino)-benzyl]-thiazolidine-2,4-dione (DN-108), 5-{[4-(2-(2,3-dihydroindol-1-yl)ethoxy)phenyl]methyl}-thiazolidine-2,4-dione, 5-[3-(4-chloro-phenyl)]-2-propynyl]-5-phenylsulfonyl)thiazolidine-2,4-dione, 5-[3-(4-chlorophenyl)]-2-propynyl]-5-(4-fluorophenyl-sulfonyl)thiazolidine-2,4-dione, 5-{[4-(2-(methyl-2-pyridinyl-amino)ethoxy)phenyl]methyl}-thiazolidine-2,4-dione (rosiglitazone), 5-{[4-(2-(5-ethyl-2-pyridyl)ethoxy)phenyl]-methyl}-thiazolidine-2,4-dione (pioglitazone), 5-{[4-((3,4-dihydro-6-hydroxy-2,5,7,8-tetramethyl-2H-1-benzopyran-2-yl)methoxy)-phenyl]-methyl}-thiazolidine-2,4-dione (troglitazone), 5-[6-(2-fluoro-benzyloxy)naphthalen-2-ylmethyl]-thiazolidine-2,4-dione (MCC-555), 5-{[2-(2-naphthyl)-benzoxazol-5-yl]-methyl}thiazolidine-2,4-dione (T-174), and 5-(2,4-dioxothiazolidin-5-ylmethyl)-2-methoxy-N-(4-trifluoromethyl-benzyl)benzamide (KRP297). In a preferred embodiment, the thiazolidine drug can contain, for example, pioglitazone, rosiglitazone, or troglitazone.

The present invention provides a combination product including an SGLT2 inhibitor and an insulin resistance improver for use in treating a subject having diabetes mellitus and lung adenocarcinoma. The SGLT2 inhibitor and the insulin resistance improver may be included in the combination product separately, or as a mixture. That is, the combination product may include a container containing the SGLT2 inhibitor and a container containing the insulin resistance improver, or a container containing the SGLT2 inhibitor and the insulin resistance improver. The combination product including a first container containing the SGLT2 inhibitor and a second container containing the insulin resistance improver may include a package containing the first container and the second container. The combination product may further include an instruction (e.g., a package insert) for administration of the SGLT2 inhibitor and the insulin resistance improver. The combination product is a pharmaceutical product, and is used for pharmaceutical applications.

The pharmaceutical composition of the present invention may be used in combination with an additional anticancer agent. Thereby, the present invention enables reduction of the amount of usage of the additional anticancer agent, leading to safer cancer treatment.

### Examples

### Example 1: Analysis of Expressions of SGLT1 and SGLT2 Using Biopsies of Lung Adenocarcinoma

In the present Example, expressions of SGLT1 and SGLT2 in biopsies or resected tissues obtained from lung adenocarcinoma patients were examined.

Among specimens of 182 cases of resection conducted in a community general hospital from October 2010 to September 2017, specimens of 102 lung adenocarcinoma cases that allowed immunostaining and clinical pathological examination were used. The mean age of the patients was 70.5 years old, and the ages of the patients ranged from 43 years old to 89 years old. The details of the patients were as shown in Table 1 below.

### [Table 1]

**Table 1: Profiles of 102 lung adenocarcinoma cases**

| | | SGLT1 ≥ 10 | | | SGLT2 ≥ 10 | | | SGLT2 ≥ 20 | | |
|---|---|---|---|---|---|---|---|---|---|---|
| Attribute | n | Positive (%) | Negative (%) | P | Positive (%) | Negative (%) | P | Positive (%) | Negative (%) | p |
| Age | | | | | | | | | | |
| 70> | 47 | 5 (10.6) | 42 | | 15 (31.9) | 32 | | 11 (23.4) | 36 | |
| 70 | 55 | 6 (10.9) | 49 | 0.965 | 20 (36.4) | 35 | 0.637 | 17 (30.9) | 38 | 0.397 |

| Sex | | | | | | | | | | |
|---|---|---|---|---|---|---|---|---|---|---|
| Female | 45 | 4 (8.9) | 41 | | 18 (40.0) | 27 | | 15 (33.3) | 30 | |
| Male | 57 | 7 (12.3) | 50 | 0.584 | 17 (29.8) | 40 | 0.283 | 13 (22.8) | 44 | 0.237 |

| Diabetes mellitus | | | | | | | | | | |
|---|---|---|---|---|---|---|---|---|---|---|
| Affected | 10 | 2 (10.0) | 8 | | 2 (20.0) | 8 | | 2 (20.0) | 8 | |
| Not affected | 92 | 10 (10.9) | 82 | 0.933 | 33 (35.9) | 59 | 0.315 | 26 (28.3) | 66 | 0.578 |

| CEA value (ng/mL) | | | | | | | | | | |
|---|---|---|---|---|---|---|---|---|---|---|
| 5> | 63 | 6 (9.5) | 57 | | 22 (34.9) | 41 | | 19 (30.2) | 44 | |
| 5≤ | 39 | 5 (12.8) | 34 | 0.602 | 13 (33.3) | 26 | 0.870 | 9 (23.1) | 30 | 0.436 |

| Smoking (BI=700) | | | | | | | | | | |
|---|---|---|---|---|---|---|---|---|---|---|
| Never and Former | 69 | 7 (10.1) | 62 | | 27 (39.1) | 42 | | 24 (34.8) | 45 | |
| Curren t | 33 | 4 (12.1) | 29 | 0.763 | 8 (24.2) | 25 | 0.138 | 4 (12.1) | 29 | 0.016 |

| EGFR mutation | | | | | | | | | | |
|---|---|---|---|---|---|---|---|---|---|---|
| With mutation | 36 | 5 (13.9) | 31 | | 14 (38.9) | 22 | | 12 (33.3) | 24 | |
| Without mutation | 42 | 4 (9.5) | 38 | | 14 (33.3) | 28 | | 11 (26.2) | 31 | |
| Unknown | 24 | 2 (8.3) | 22 | 0.748 | 7 (29.2) | 17 | 0.728 | 5 (20.8) | 19 | 0.553 |

| Stage | | | | | | | | | | |
|---|---|---|---|---|---|---|---|---|---|---|
| I-II | 79 | 8 (10.1) | 71 | | 29 (36.7) | 50 | | 24 (30.4) | 55 | |
| III-IV | 23 | 3 (13.0) | 20 | 0.691 | 6 (26.1) | 17 | 0.345 | 4 (17.4) | 19 | 0.219 |

| SGLT1 | | | | | | | | | | |
|---|---|---|---|---|---|---|---|---|---|---|
| SGLT1 ≥ 10 | 11 | | | | 3 (27.3) | 8 | | 3 (27.3) | 8 | |
| SGLT1 < 10 | 91 | | | | 32 (35.2) | 59 | 0.603 | 25 (27.5) | 66 | 0.989 |
| Total | 10 2 | 11 (100) | 91 | | 35 (100) | 67 | | 28 (100) | 74 | |

In Table 1, "DM" indicates diabetes mellitus, "CEA" indicates expression of a CEA marker, and "EGFR" indicates epidermal growth factor receptor. The EGFR mutation is any driver mutation of G719X, deletion in exon 19 (del19), S768I, insertion in exon 20, T790M, L858R, and L861Q.

Tissue sections were produced from biopsies or tissue specimens according to a conventional method, and subjected to immunohistochemical staining. An anti-SGLT1 antibody (Abeam plc.: 1/500) and an anti-SGLT2 antibody (Abeam plc.: 1/180) were used as primary antibodies. A rabbit anti-mouse IgG antibody included in a BOND Polymer Refine Detection (Leica Biosystems Nussloch GmbH) kit was used as a secondary antibody according to an instruction provided by the manufacturer. Thereafter, a horseradish peroxidase (HRP)-labeled anti-rabbit IgG antibody was used for staining. The staining was performed by using the automated immunostainer BOND-MAX (Leica Biosystems Nussloch GmbH). Evaluation of stained images was carried out by using virtual slides (apparatus used: Nano Zoomer-XR (HAMAMATSU PHOTONICS K.K.), conditions: 20× mode, Single Layer).

Expressions of SGLT1 and SGLT2 were evaluated by using two cutoffs for each. Specifically, a cutoff such that a case that expression was found in 10% or more of the cells of a cancer cell population in a section was regarded as being positive (10% cutoff) and a cutoff such that a case that expression was found in 20% or more of the cells of a cancer cell population in a section was regarded as being positive (20% cutoff) were used for evaluation.

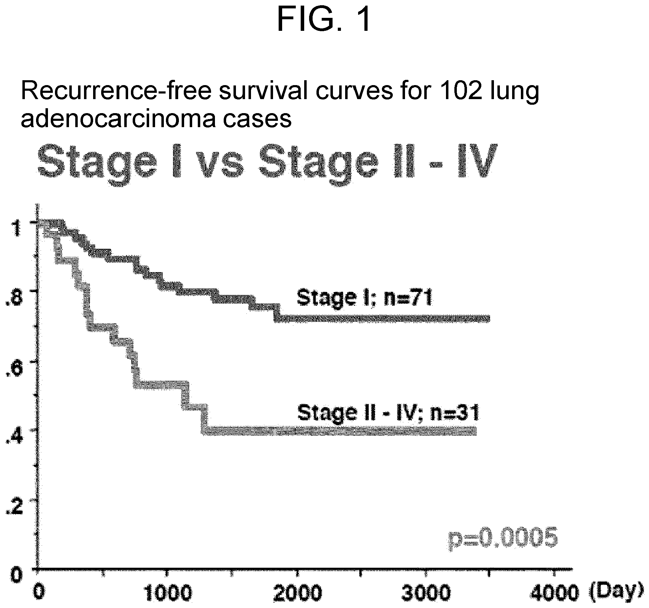

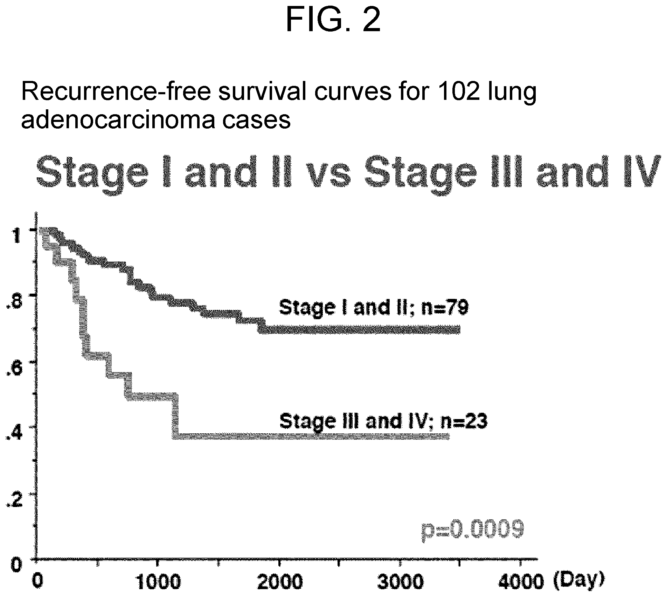

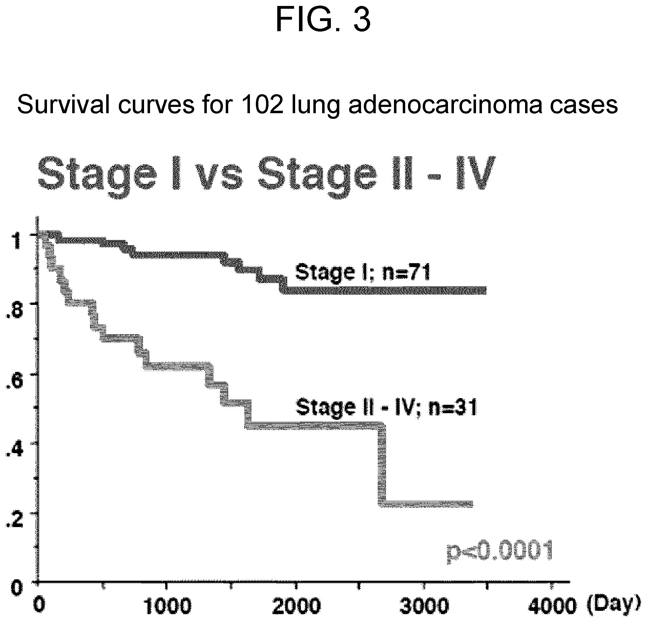

As demonstrated in Figure 1, comparison between Stage I and Stage II found a tendency that the recurrence-free survival time for Stage I was longer than that for Stage II. As demonstrated in Figure 2, comparison between Stage I and II and Stage III and IV found a tendency that the recurrence-free survival time for early-stage cancer (Stage I and II) was longer than that for Stage III and IV. As demonstrated in Figure 3, comparison between the survival curve for Stage I and that for Stage II to IV found that the number of long-surviving patients for Stage I was larger than that for other stages. This was so also for comparison between Stage I and II and Stage III and IV, showing that the number of long-surviving patients for early-stage cancer (Stage I and II) was larger than that for Stage III and IV.

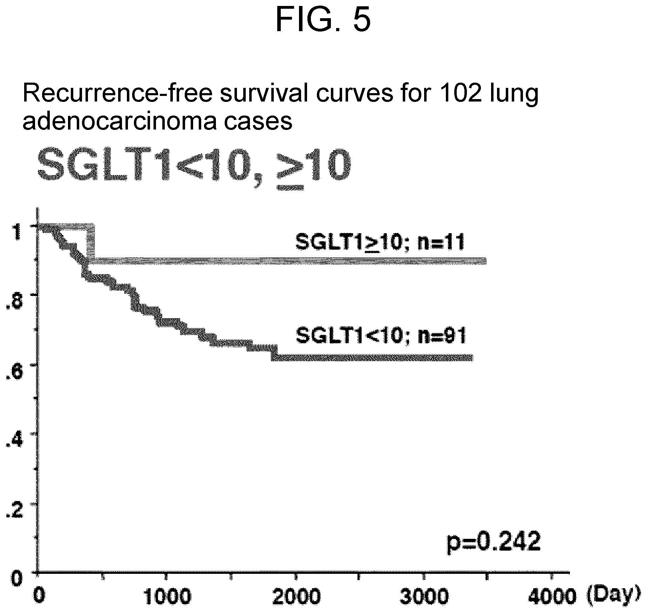

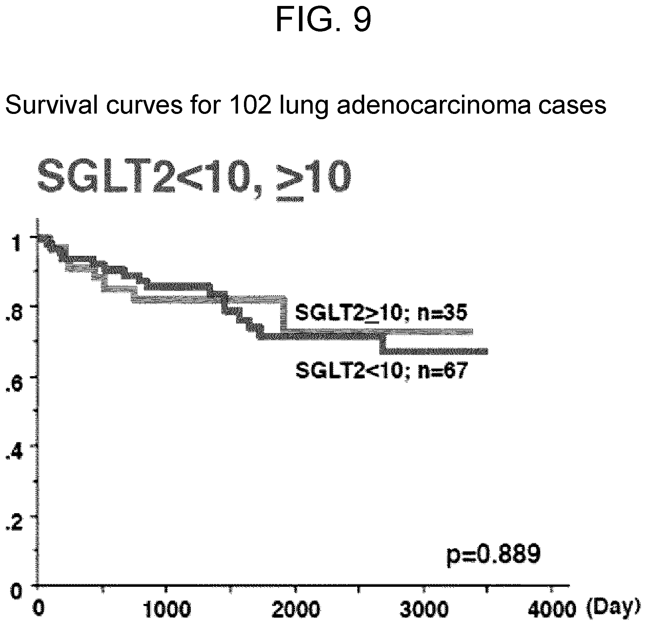

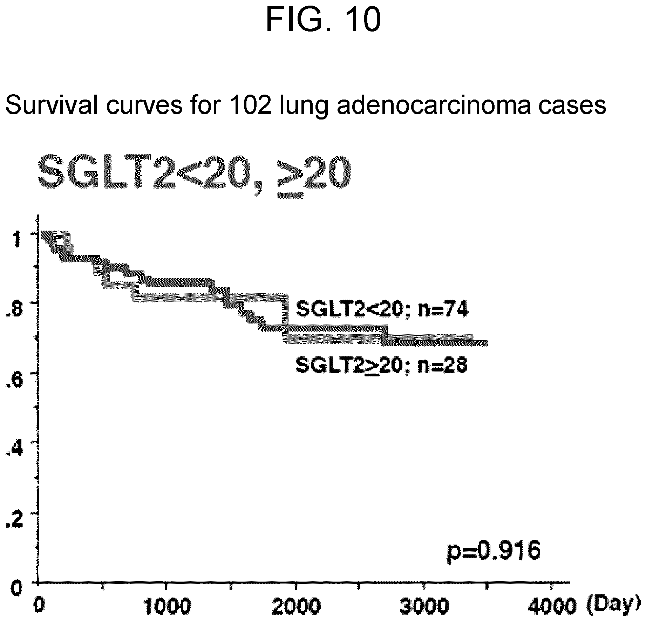

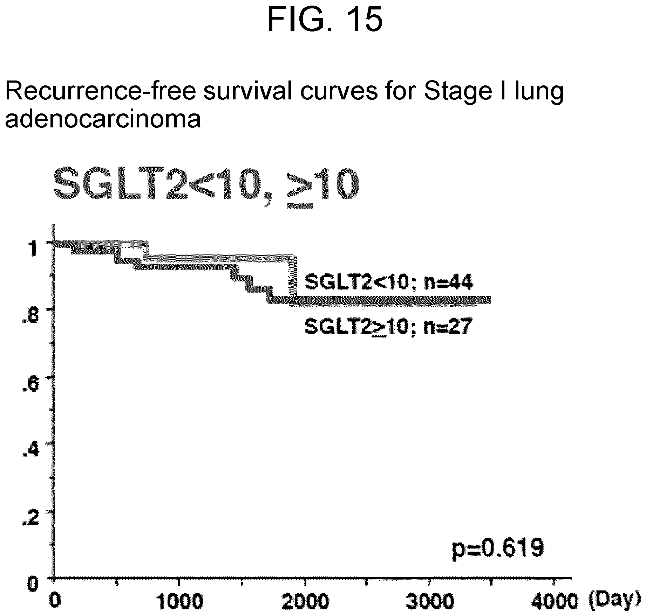

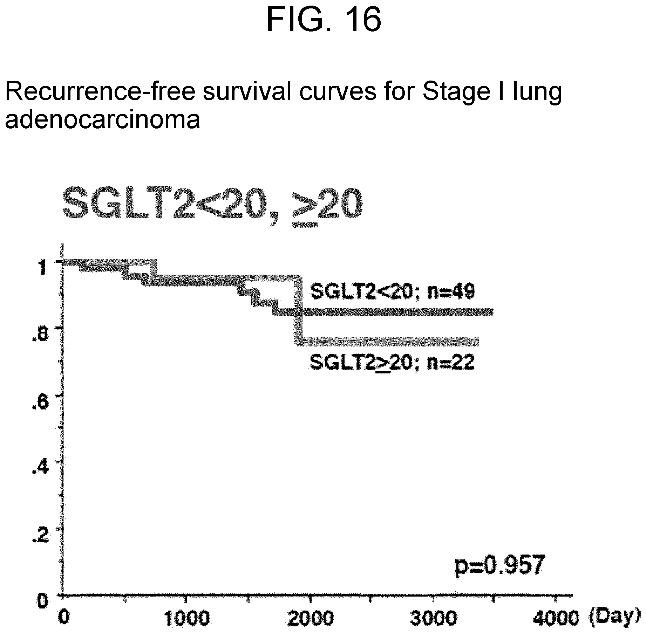

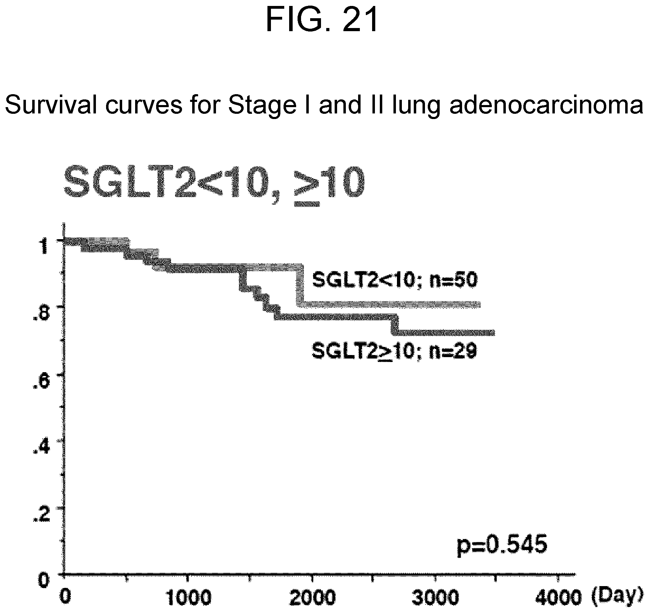

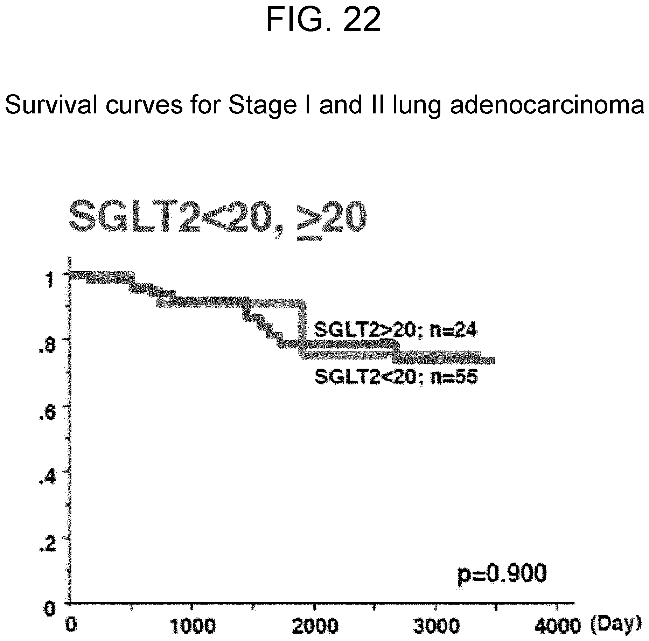

Next, the relationship between expressions of SGLT1 and SGLT2 and the recurrence-free survival time or survival curve was examined for the patients in Stage I to IV or patients with early-stage cancer (Stage I and II: 79 patients in total). The results were as shown in Figures 5 to 22. The results shown in Figures 5 to 22 are summarized in Table 2.

### [Table 2]

**Table 2: Results on relationship between SGLT1 and SGLT2 and recurrence-free survival time or survival curve**

| DFI (recurrence-free survival) | Stage I-IV (n=102) | Stage I (n=71) | Stage I and II (n=79) |
|---|---|---|---|
| SGLT1 ≥10 | Figure 5 | Figure 11 | Figure 17 |
| SGLT2 ≥10 | Figure 6 | Figure 12 | Figure 18 |
| SGLT2 ≥20 | Figure 7 | Figure 13 | Figure 19 |
| | | | |

| Survival rate | Stage I-IV (n=102) | Stage I (n=71) | Stage I and II (n=79) |
|---|---|---|---|
| SGLT1 ≥10 | Figure 8 | Figure 14 | Figure 20 |
| SGLT2 ≥10 | Figure 9 | Figure 15 | Figure 21 |
| SGLT2 ≥20 | Figure 10 | Figure 16 | Figure 22 |

Found as shown in Figures 5 to 22 was a tendency that groups with higher expression of SGLT1 were less likely to undergo recurrence and had longer survival time than groups with lower expression of SGLT1. By contrast, found was a tendency that groups with lower expression of SGLT2 were less likely to undergo recurrence and had longer survival time than groups with higher expression of SGLT2.

Found from Figure 5 was a tendency that higher expression of SGLT1 was accompanied by longer recurrence-free survival time for 102 lung adenocarcinoma cases.

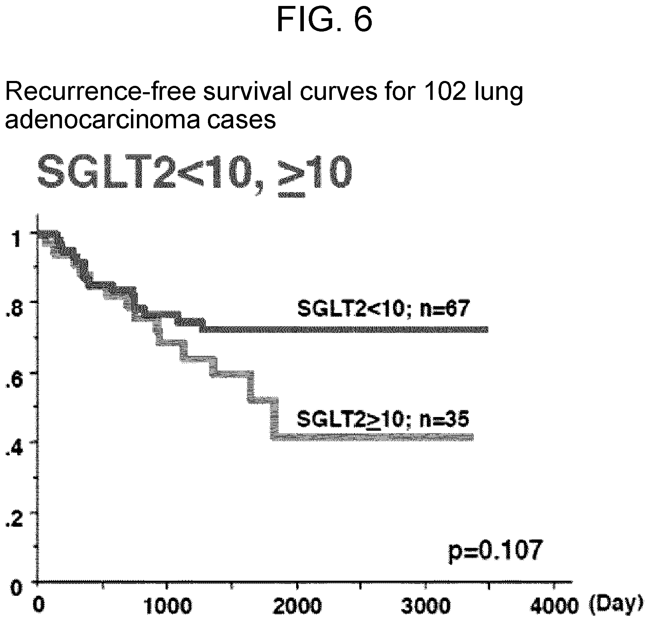

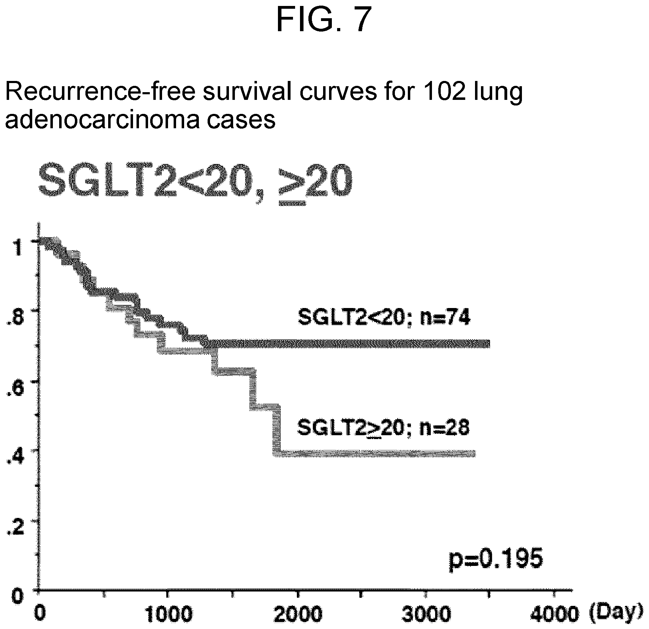

Found from Figures 6 and 7 was a tendency that lower expression of SGLT2 was accompanied by longer recurrence-free survival time.

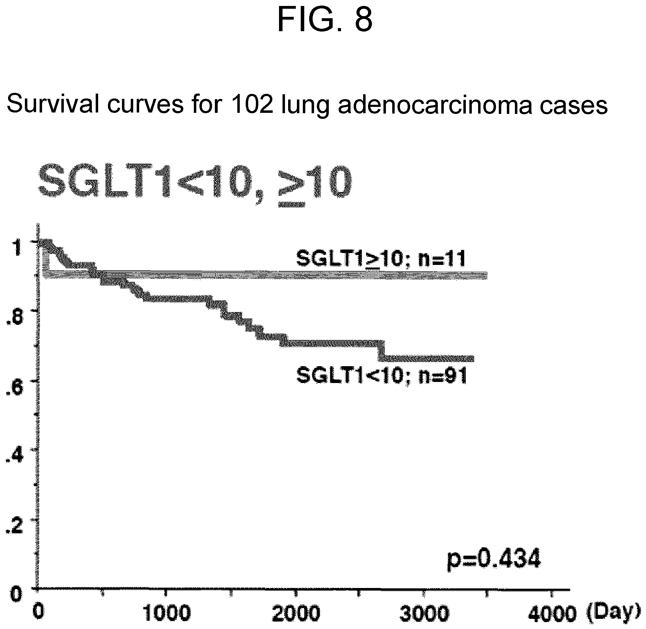

Found from Figure 8 was a tendency that the higher the expression of SGLT1, the higher the survival rate of patients.

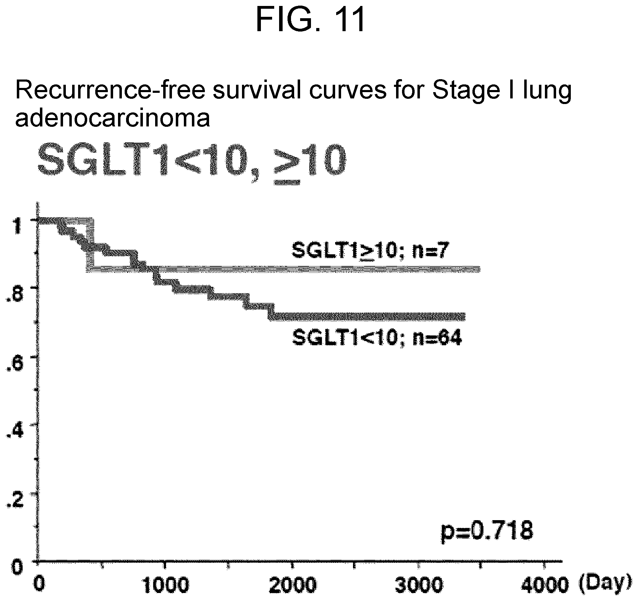

Found from Figure 11 was a tendency that higher expression of SGLT1 was accompanied by longer recurrence-free survival time for Stage I lung adenocarcinoma.

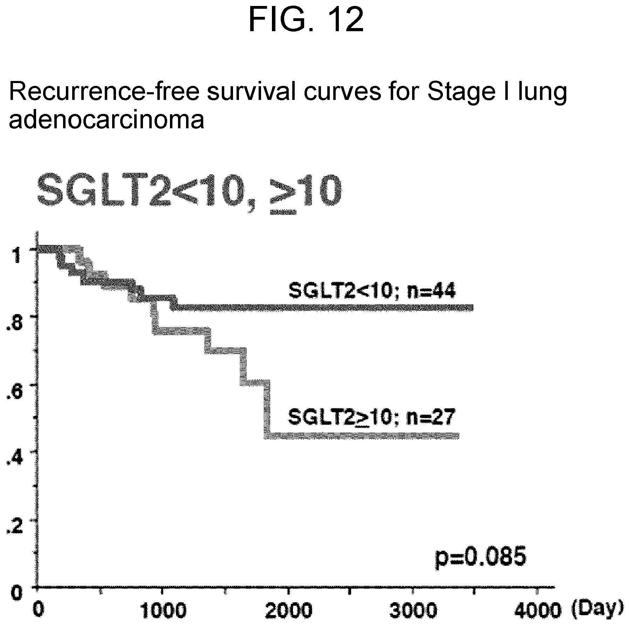

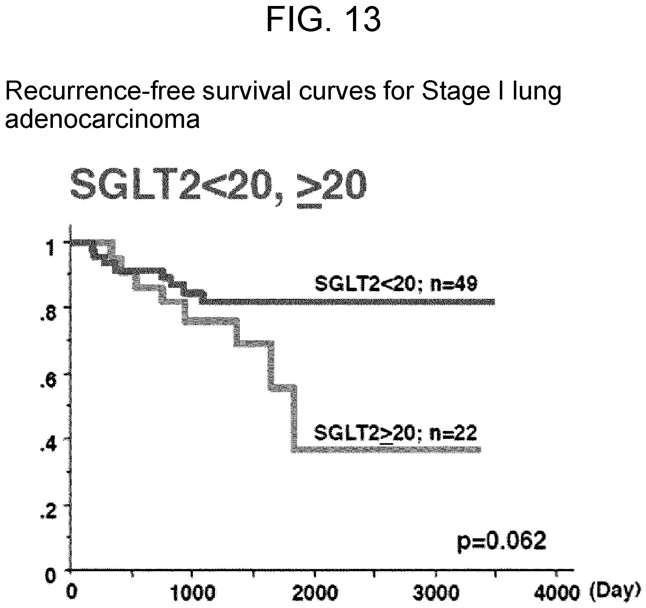

Found from Figures 12 and 13 was a tendency that lower expression of SGLT2 was accompanied by longer recurrence-free survival time for Stage I lung adenocarcinoma.

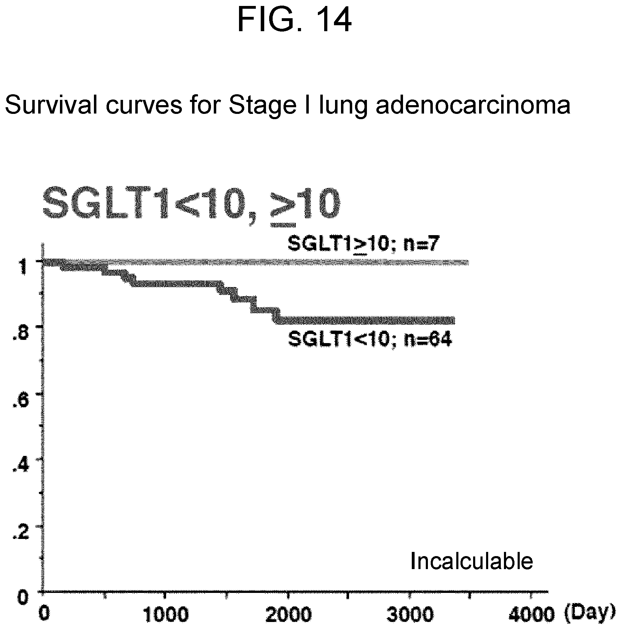

Found as shown in Figure 14 was a tendency that higher expression of SGLT1 was accompanied by higher survival rates for Stage I lung adenocarcinoma.

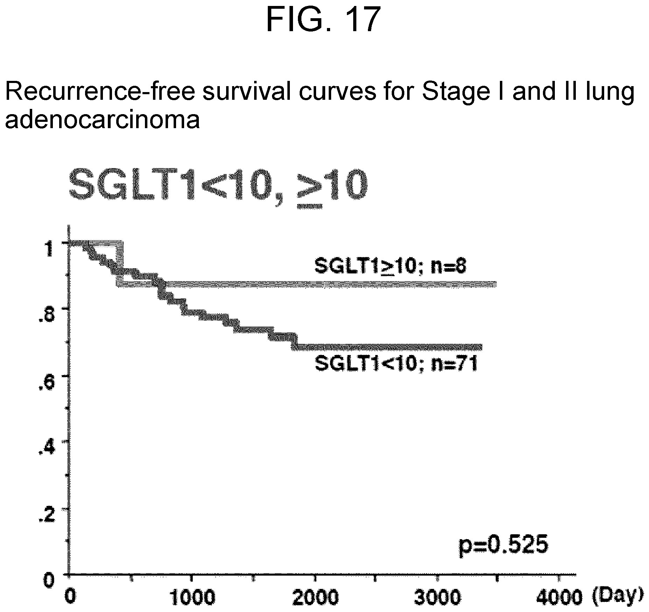

Found as shown in Figure 17 was a tendency that higher expression of SGLT1 was accompanied by longer recurrence-free survival time for early-stage cancer (Stage I and II).

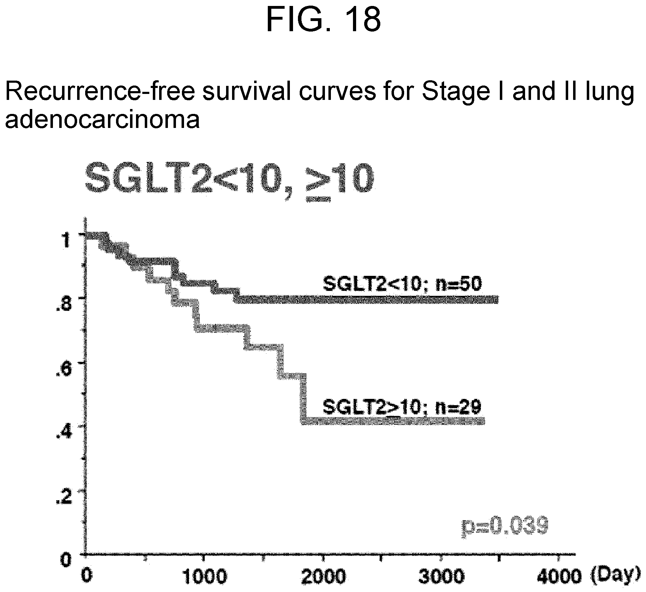

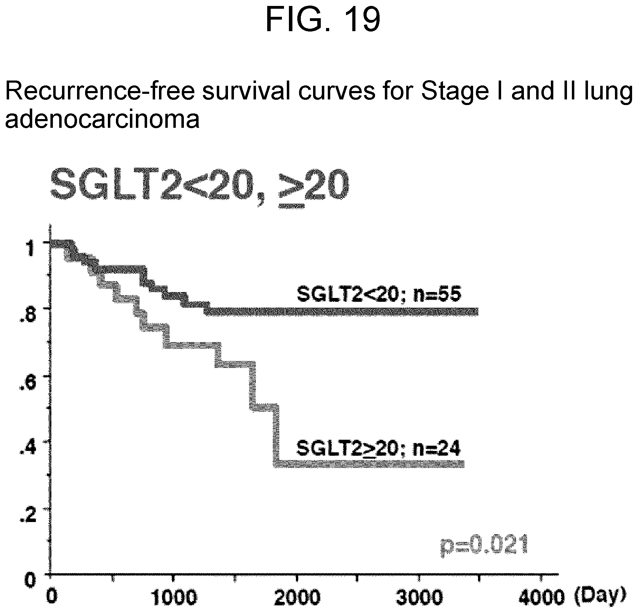

Found as shown in Figures 18 and 19 was a tendency that lower expression of SGLT2 was accompanied by longer recurrence-free survival time for early-stage cancer (Stage I and II).

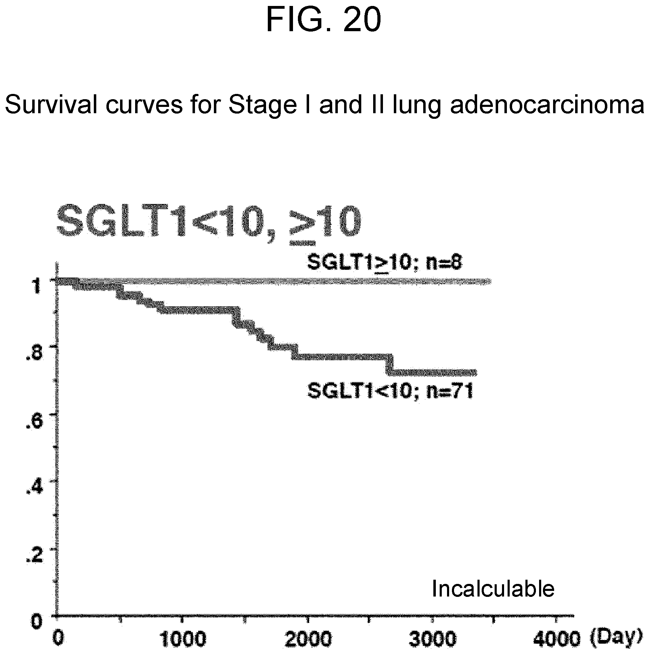

Found as shown in Figure 20 was a tendency that higher expression of SGLT1 was accompanied by higher survival rates for Stage I lung adenocarcinoma.

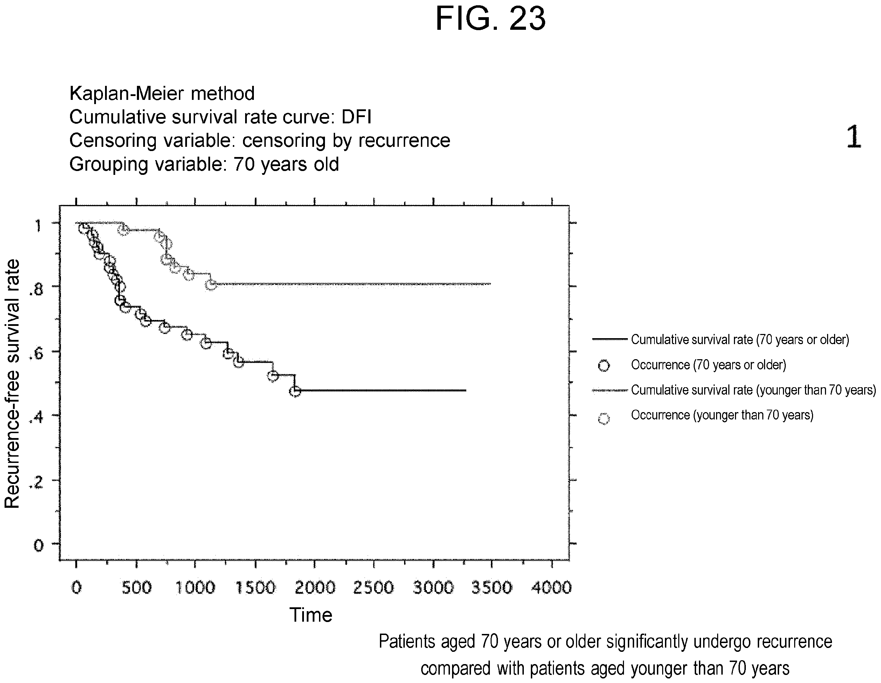

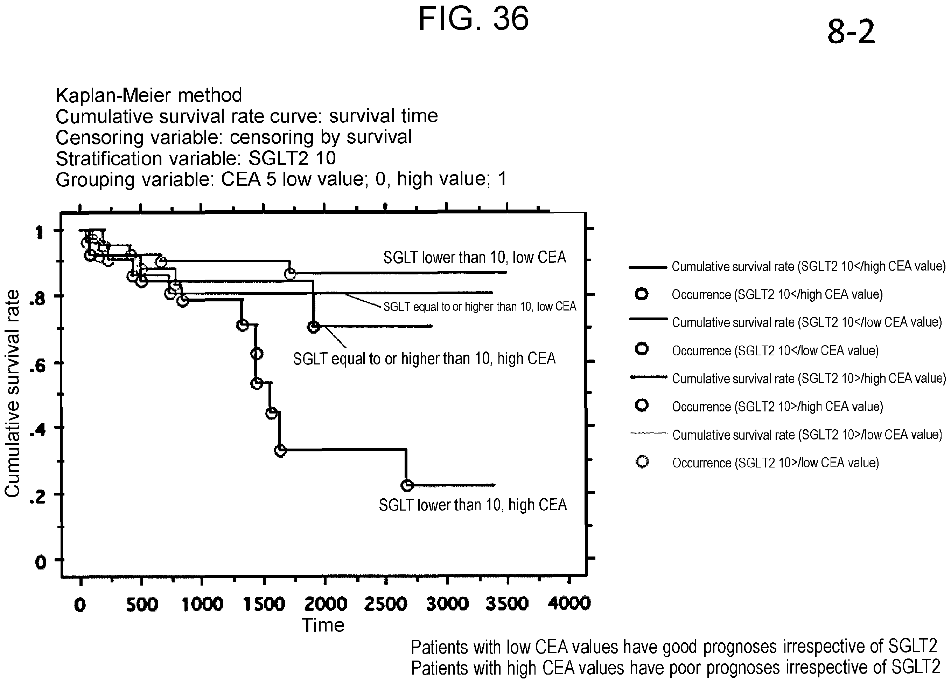

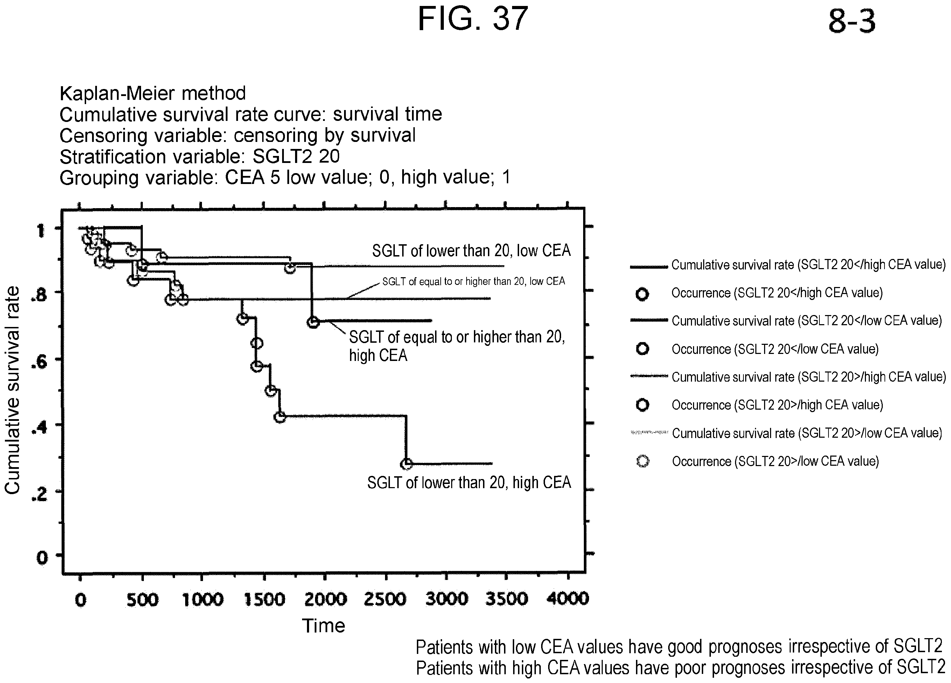

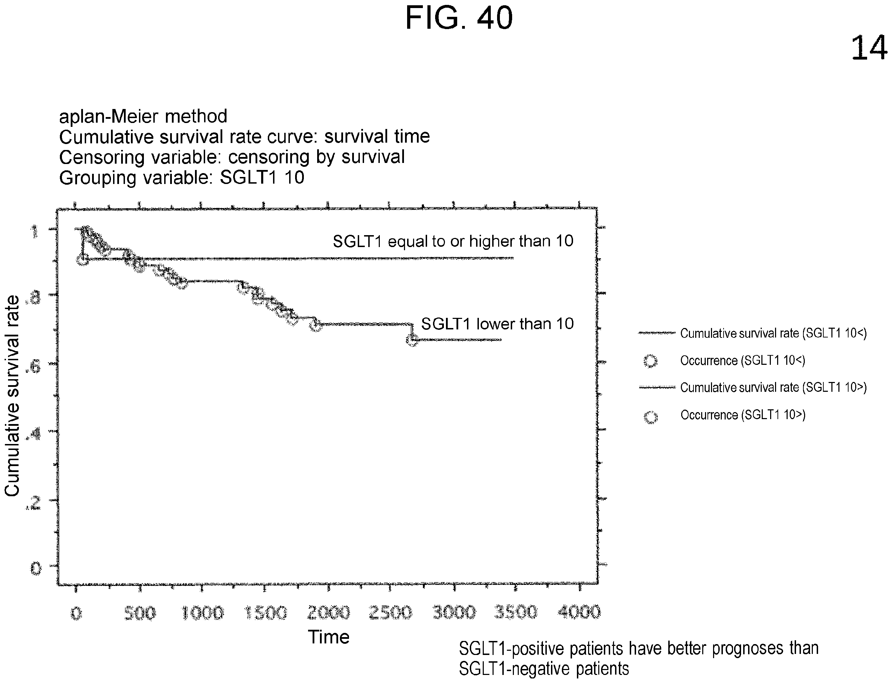

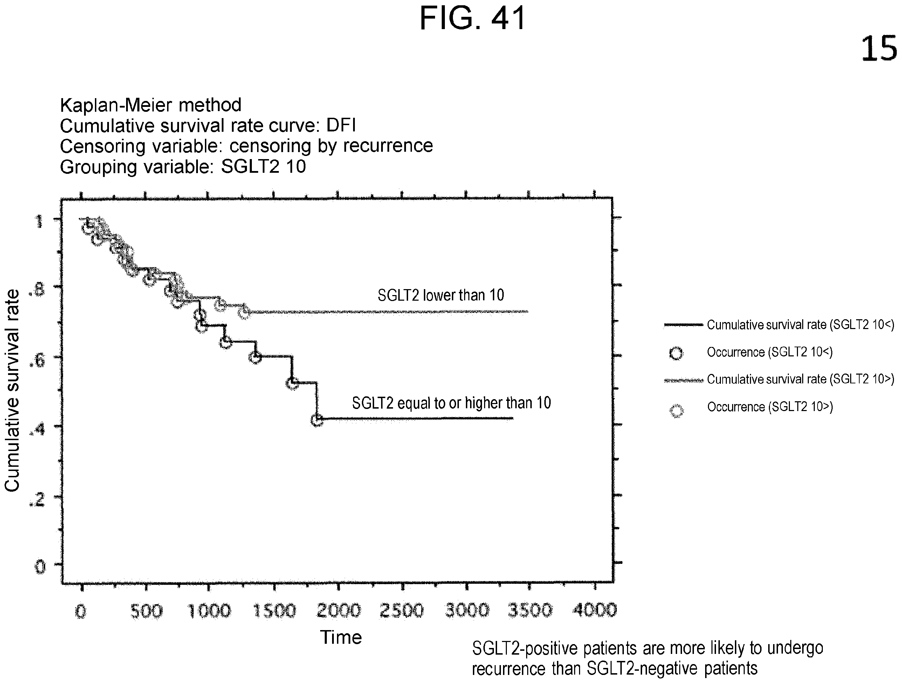

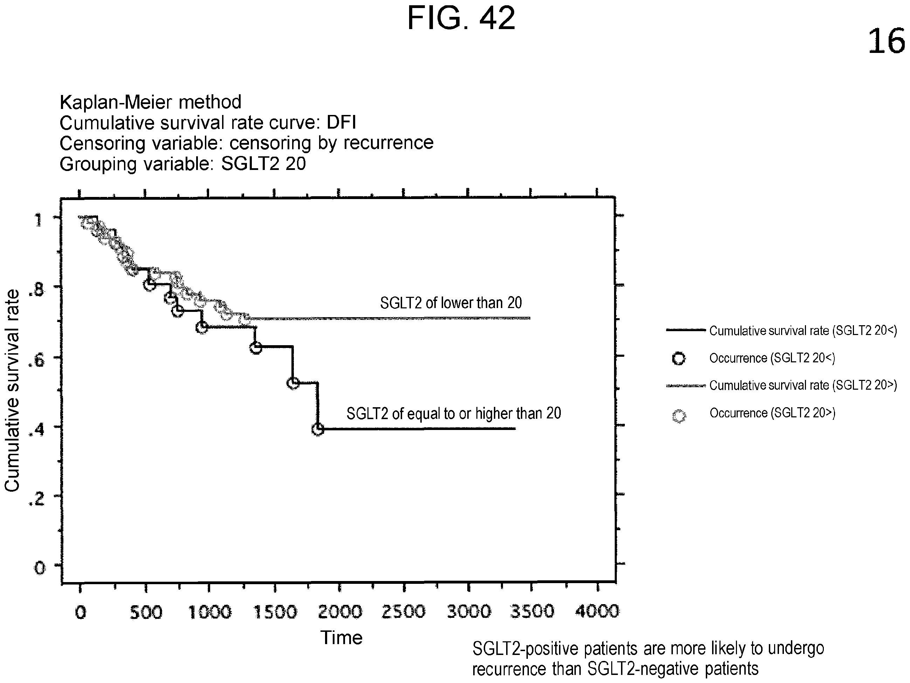

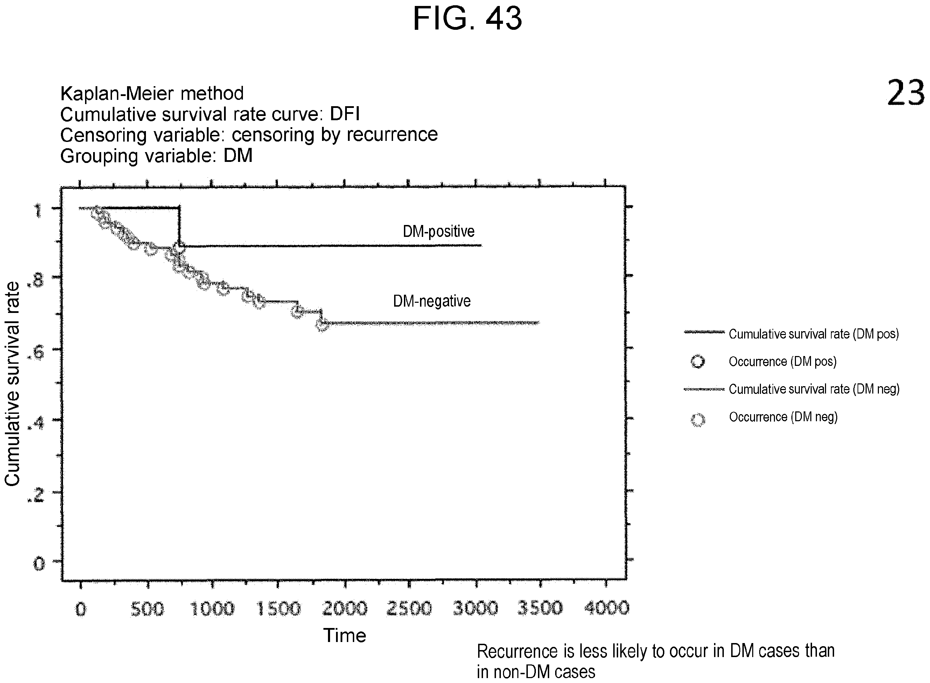

Furthermore, comparison was performed for age (70 years or older and younger than 70 years), history of diabetes mellitus (with and without DM), CEA values (high and low), and EGFR mutations (with and without any EGFR mutation). The results were as shown in Figures 23 to 44.

Found as shown in Figure 23 was a tendency that patients aged 70 years or older were more likely to undergo recurrence than patients aged younger than 70 years.

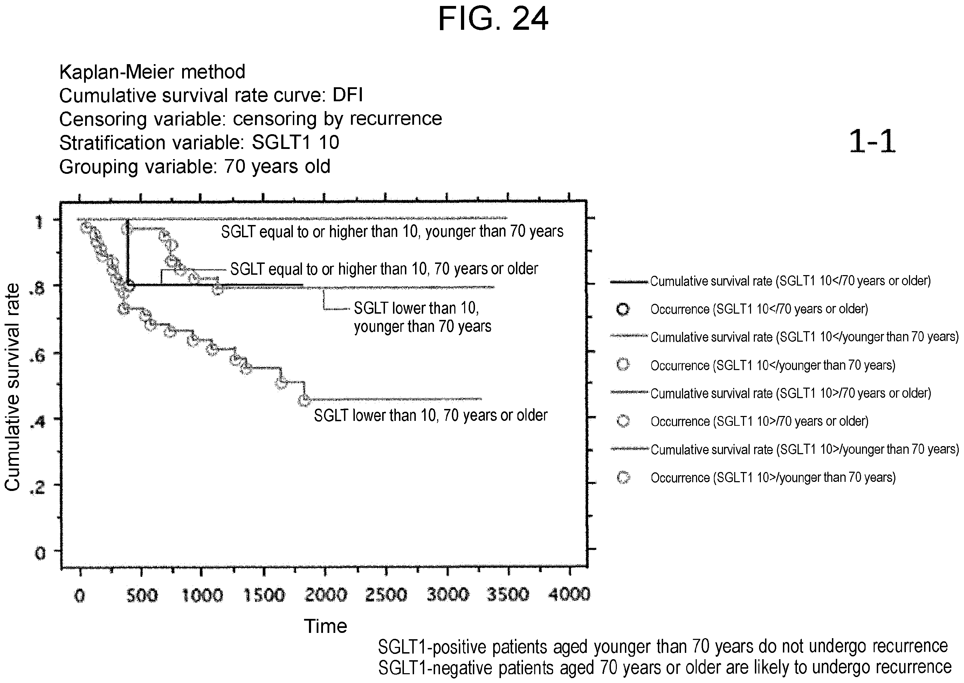

Found as shown in Figure 24 was a tendency that SGLT1-positive patients aged younger than 70 years did not undergo recurrence (were less likely to undergo recurrence) and SGLT1-negative patients aged 70 years or older were likely to undergo recurrence.

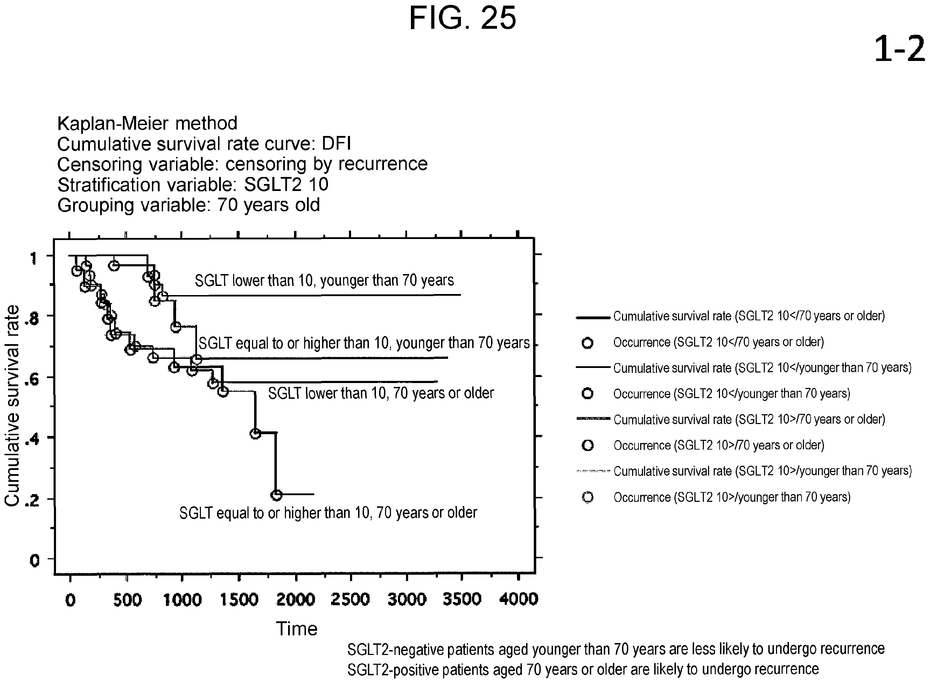

Found as shown in Figure 25 was a tendency that SGLT2-negative patients aged younger than 70 years were less likely to undergo recurrence and SGLT2-positive patients aged 70 years or older were likely to undergo recurrence.

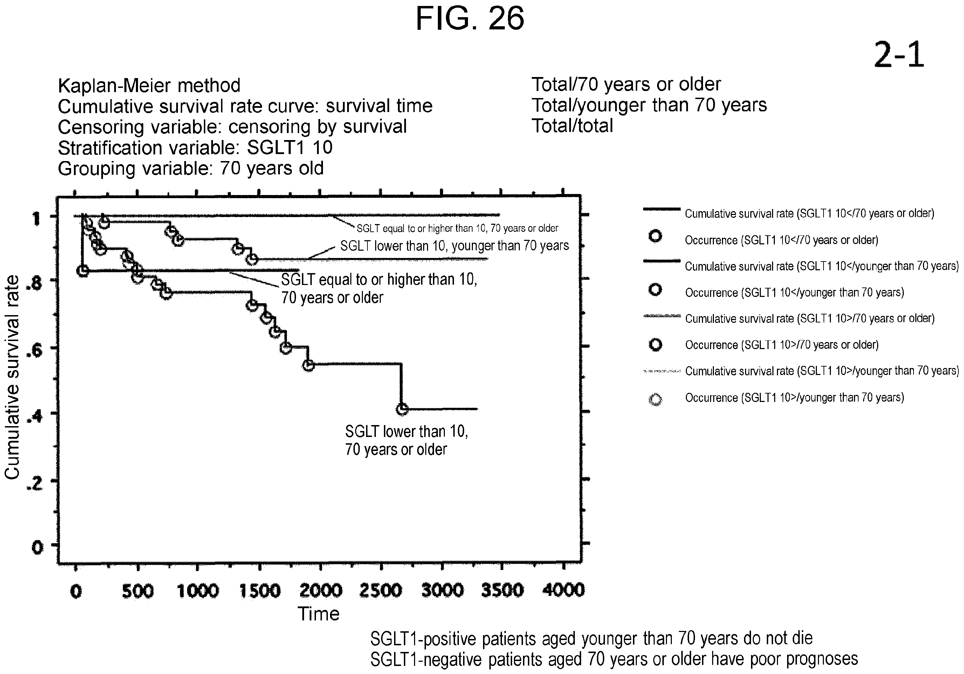

Found as shown in Figure 26 were no case of death for SGLT1-positive patients aged younger than 70 years and a tendency that SGLT1-negative patients aged 70 years or older had poor prognoses.

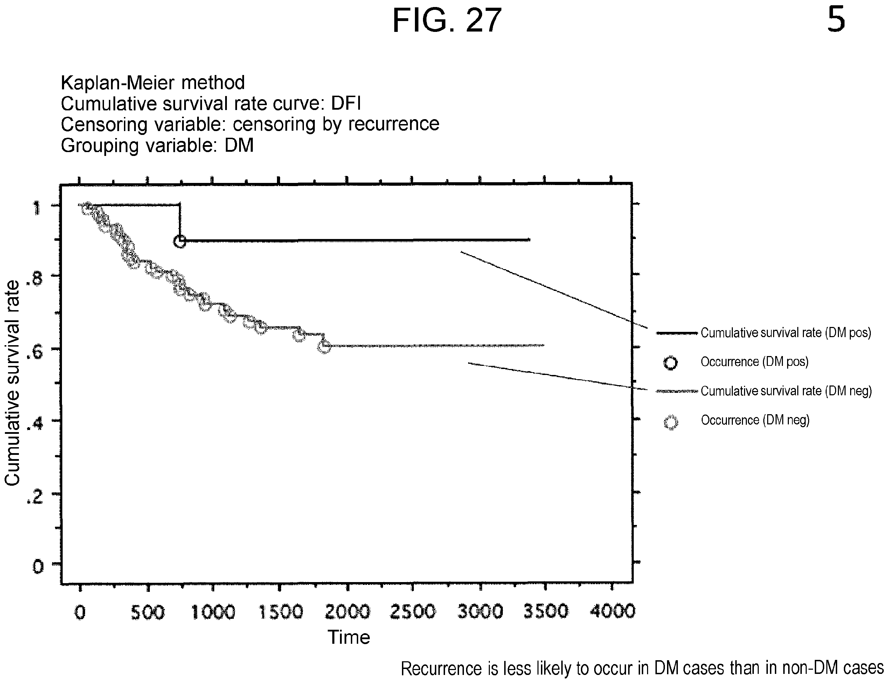

Found as shown in Figure 27 was a tendency that diabetes mellitus patients (DM) were less likely to undergo recurrence than non-diabetes mellitus patients.

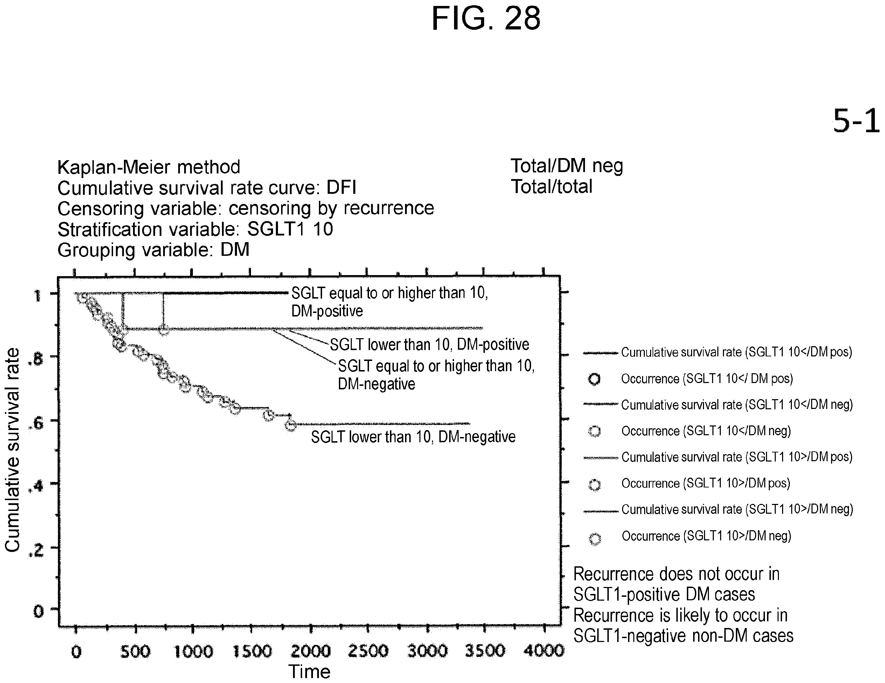

Found as shown in Figure 28 was a tendency that SGLT1-positive diabetes mellitus patients did not undergo recurrence (were less likely to undergo recurrence) and SGLT1-negative non-diabetes mellitus patients were likely to undergo recurrence.

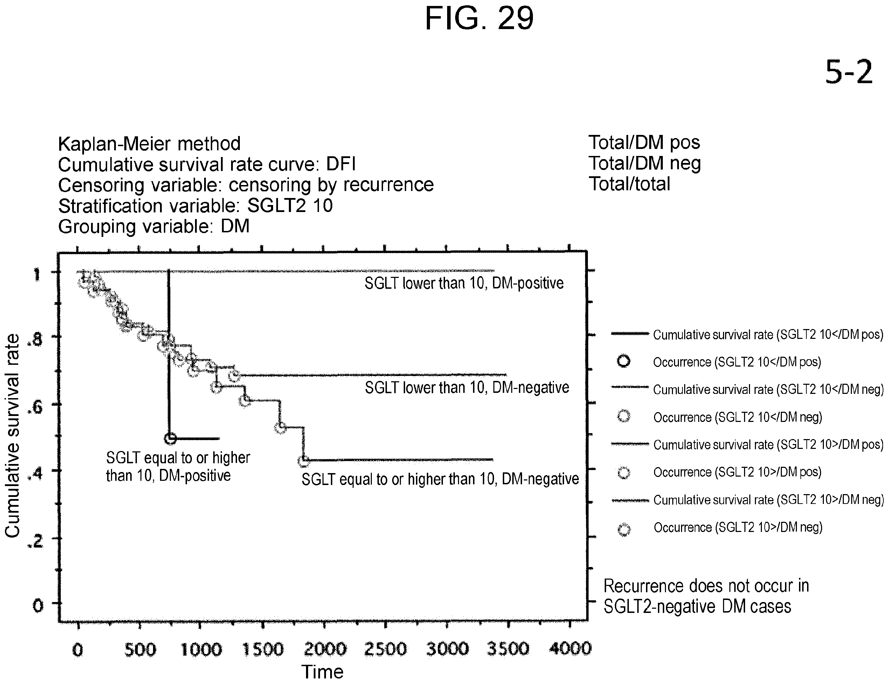

Found as shown in Figure 29 was a tendency that SGLT2-negative diabetes mellitus patients did not undergo recurrence (were less likely to undergo recurrence).

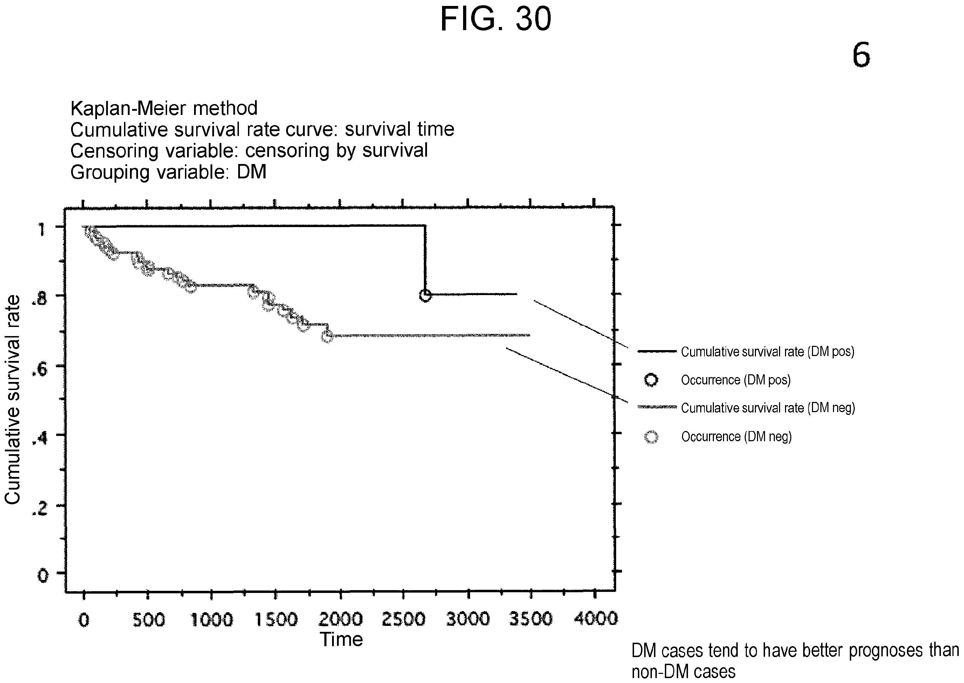

Found as shown in Figure 30 was a tendency that diabetes mellitus patients had better prognoses than non-diabetes mellitus patients.

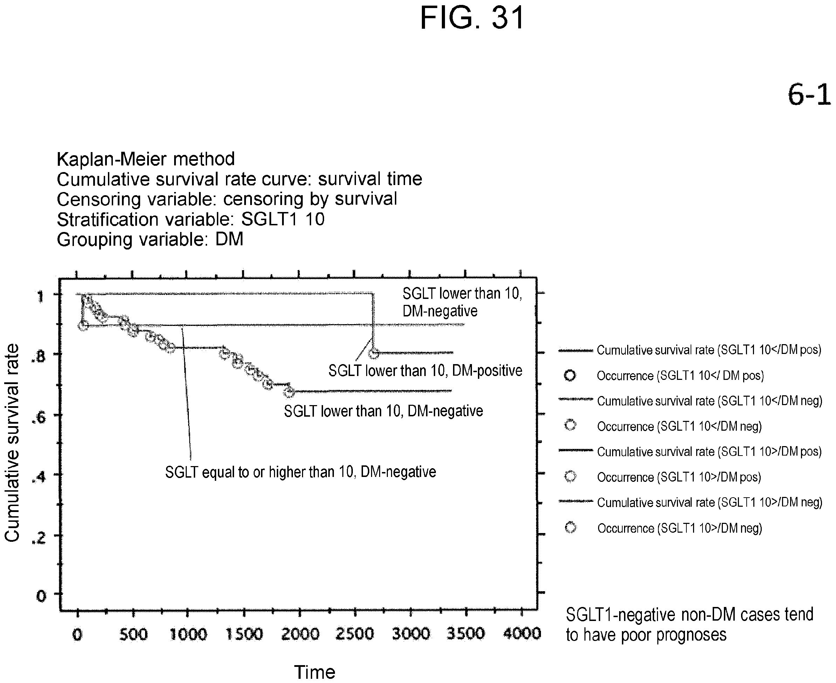

Found as shown in Figure 31 was a tendency that SGLT1-negative non-diabetes mellitus patients had poor prognoses.

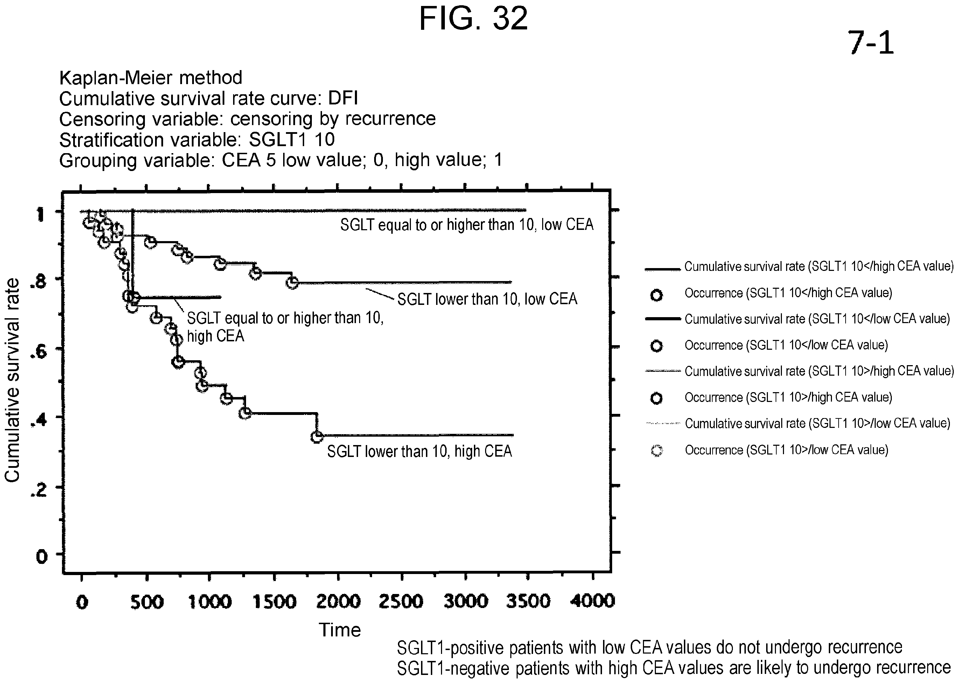

Found as shown in Figure 32 was a tendency that recurrence did not occur (was less likely to occur) in SGLT1-positive cases with low CEA values (less than 5.0 ng/ml) and recurrence was likely to occur in SGLT1-negative cases with high CEA (5.0 ng/ml or more).

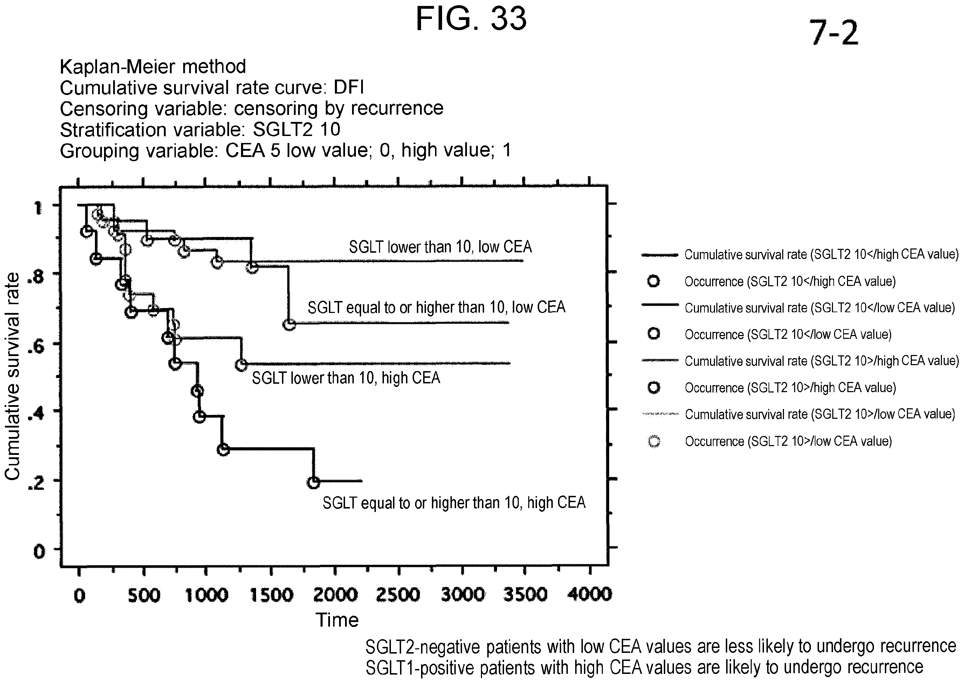

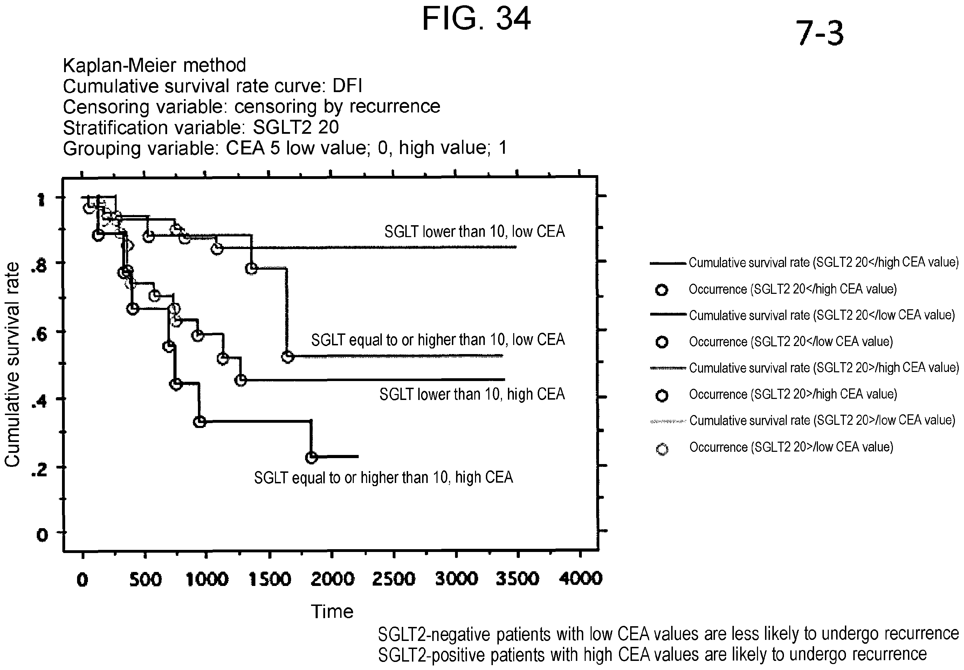

Found as shown in Figures 33 and 34 was a tendency that recurrence was less likely to occur in SGLT2-negative cases with low CEA values and recurrence was likely to occur in SGLT2-positive cases with high CEA values.

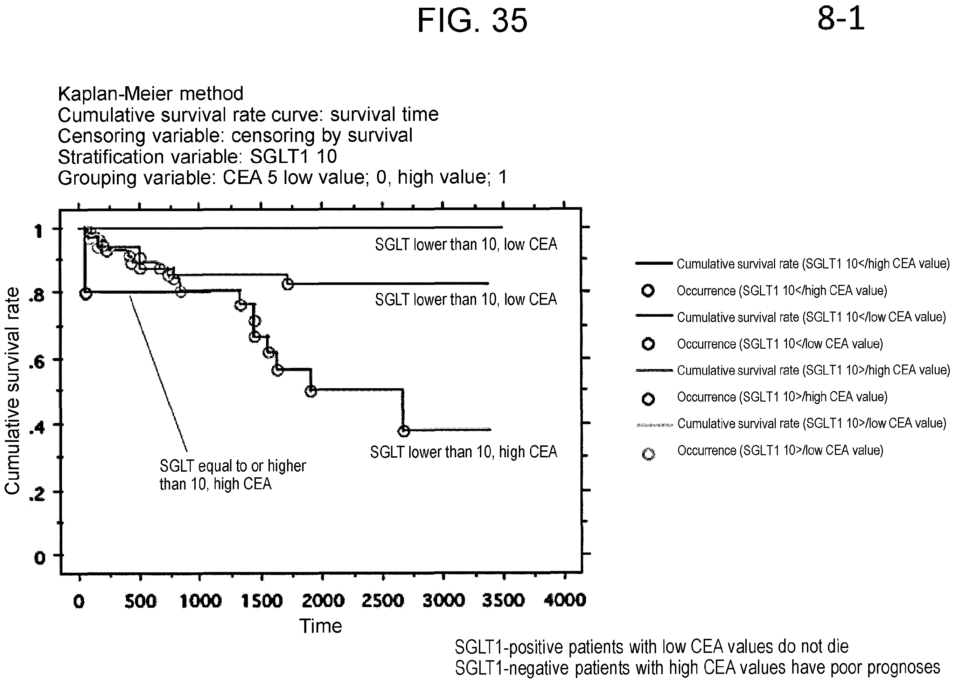

Found as shown in Figure 35 were no case of death for SGLT1-positive cases with low CEA values and a tendency that SGLT1-negative cases with high CEA values exhibited poor prognoses.

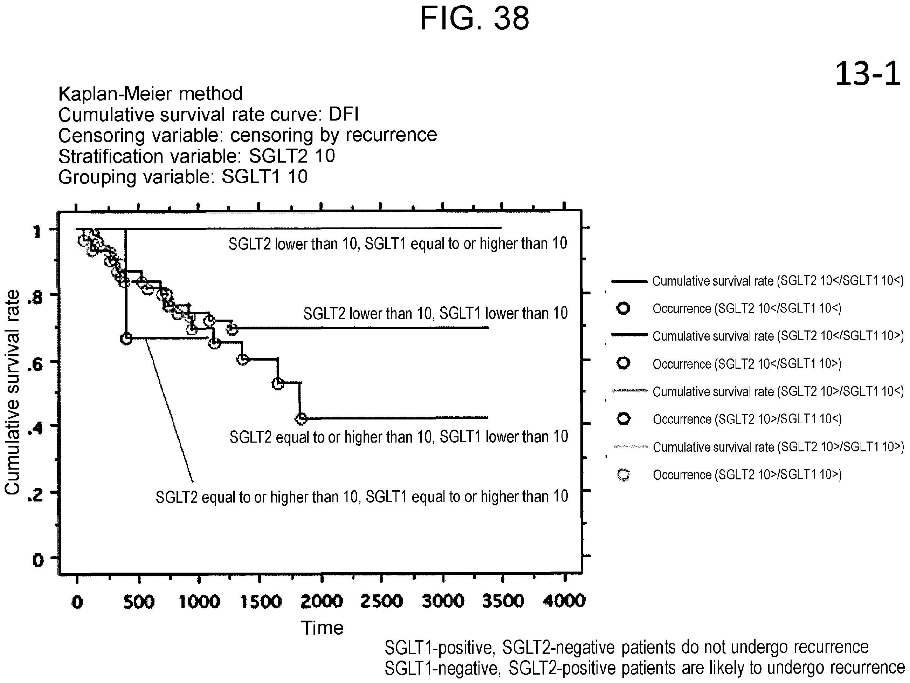

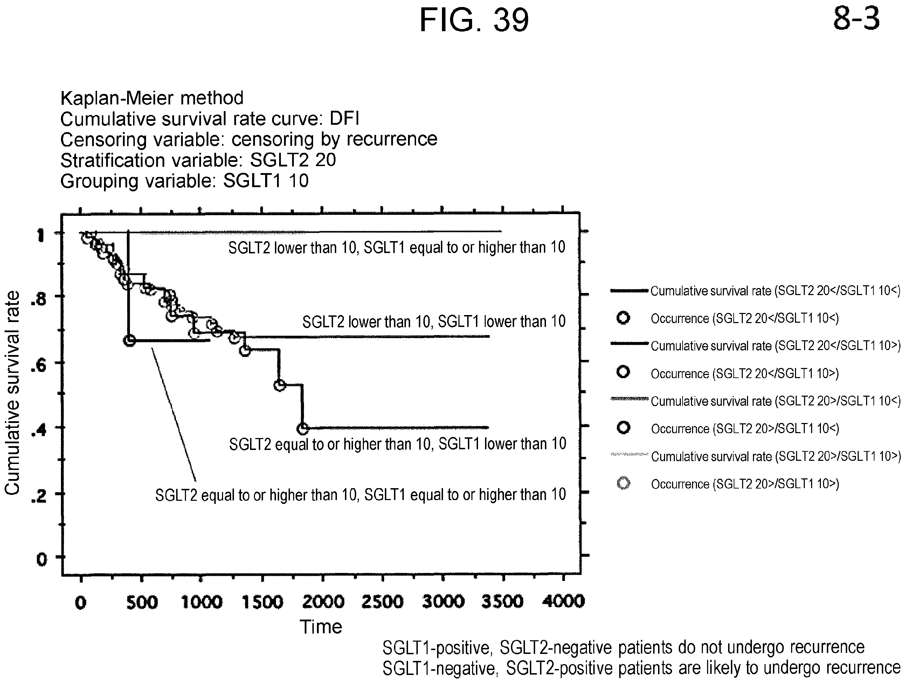

Found as shown in Figures 38 and 39 was a tendency that recurrence did not occur (was less likely to occur) in SGLT1-positive, SGLT2-negative cases and recurrence was likely to occur in SGLT1-negative, SGLT2-positive cases.

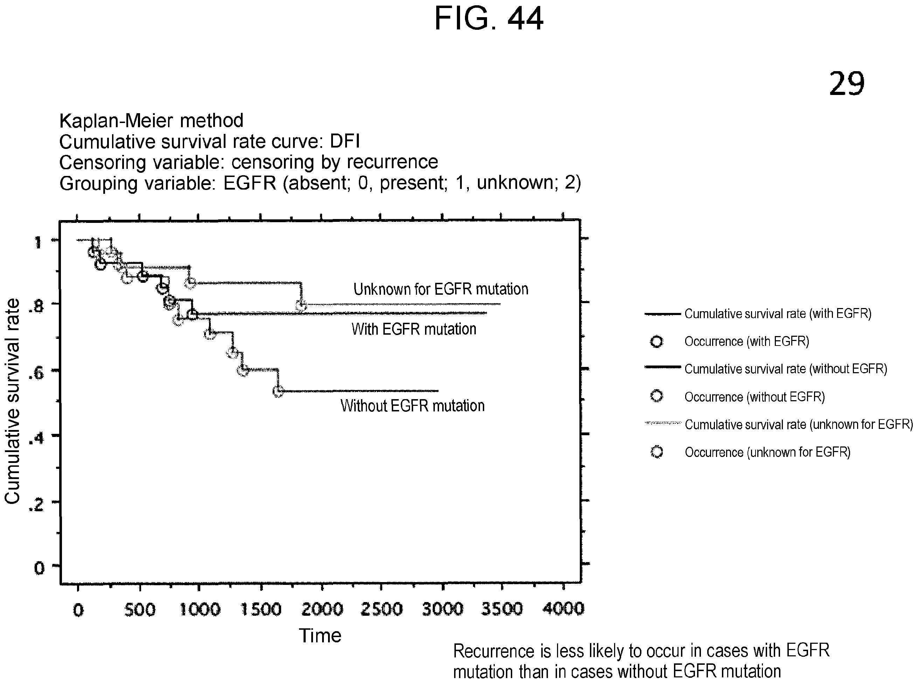

Found as shown in Figure 44 was a tendency that recurrence was less likely to occur in cases with EGFR mutation than in cases without such mutation. The EGFR mutation mentioned here is a driver mutation, and each EGFR mutation was any mutation of G719X, deletion in exon 19 (del19), S768I, insertion in exon 20, T790M, L858R, and L861Q.

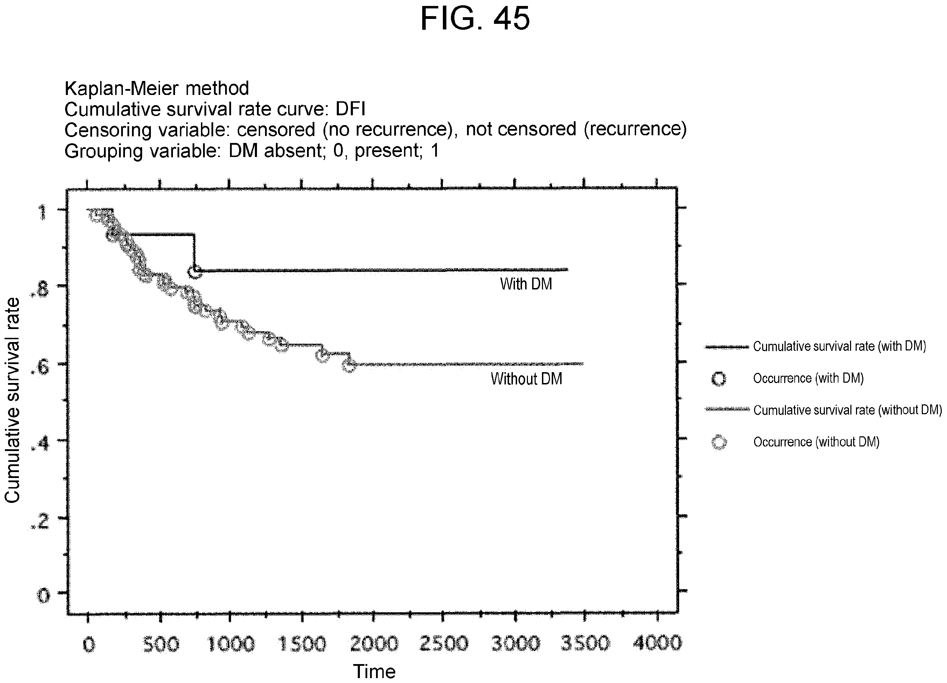

Found as shown in Figure 45 was a tendency that diabetes mellitus patients had longer recurrence-free survival time than non-diabetes mellitus patients.

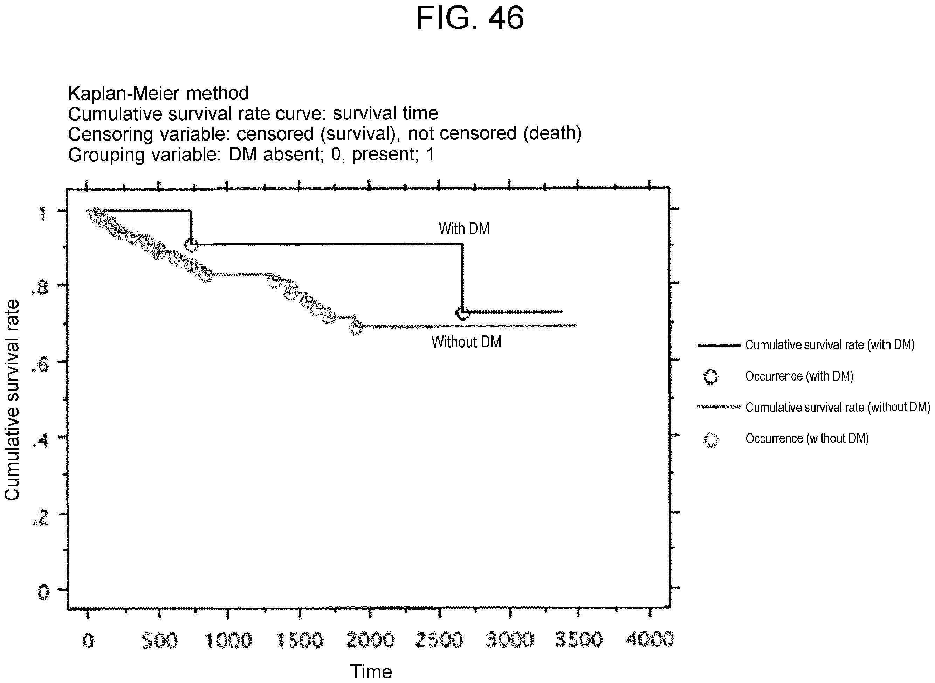

Found as shown in Figure 46 was a tendency that diabetes mellitus patients had better survival rates than non-diabetes mellitus patients.

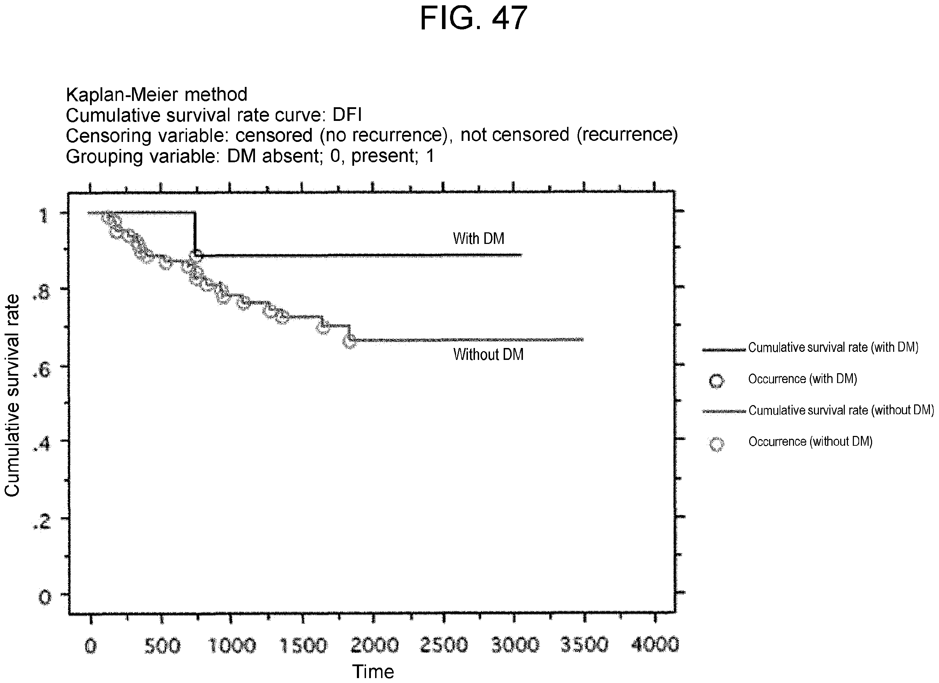

Found as shown in Figure 47 was a tendency that diabetes mellitus patients had longer recurrence-free survival time than non-diabetes mellitus patients for early-stage cancer in Stage I and II.

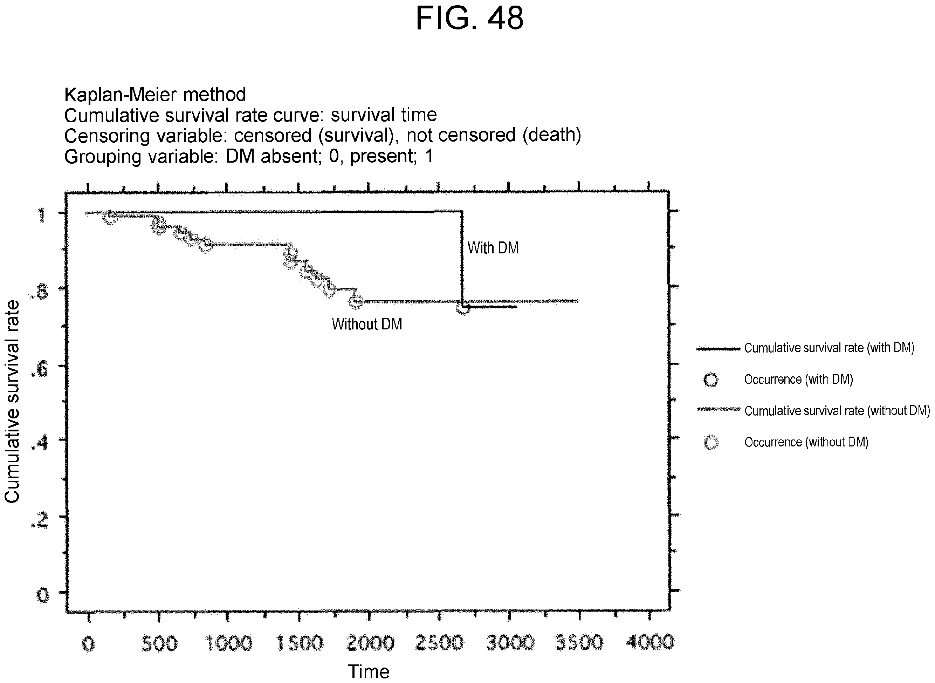

Found as shown in Figure 48 was a tendency that diabetes mellitus patients had better survival rates than non-diabetes mellitus patients for early-stage cancer in Stage I and II.

Revealed as described above was a tendency that SGLT1 correlated to both recurrence-free survival time and survival rates, and groups with higher expression of SGLT1 had longer recurrence-free survival time and higher survival rates than groups with lower expression of SGLT1. Additionally revealed was a tendency that SGLT2 correlated with recurrence-free survival time, and groups with lower expression of SGLT2 had longer recurrence-free survival time than groups with higher expression of SGLT2. Moreover revealed was a tendency that older groups had poor prognoses. Furthermore revealed was a tendency that diabetes mellitus patients were less likely to undergo recurrence and had better prognoses than non-diabetes mellitus patients. Furthermore revealed was a tendency that cases with EGFR mutation exhibited better prognoses than cases without such mutation. The cases with recurrence included cases with primary recurrence and cases with metastasis, and the same tendency was found for primary recurrence and metastasis.

No relationship was found between prognoses and sex. Smoking (BI index: 700 or higher, smoking had been continued before hospitalization) adversely influenced prognoses, no recurrence was found in SGLT1-positive non-smoking cases, and a tendency that recurrence was likely to occur in SGLT1-negative smoking cases was found. Recurrence was less likely to occur in SGLT2-negative non-smoking cases, and recurrence occurred in all the SGLT2-positive smoking cases.

The reason why diabetes mellitus patients had better prognoses than non-diabetes mellitus patients is presumably that some of the diabetes mellitus patients were subjected to SGLT2 inhibition therapy, metformin therapy, DPP-4 inhibitor therapy, or the like. The reason why cases with EGFR mutation exhibited better prognoses than cases without such mutation is presumably that the cases with EGFR mutation were under EGFR-targeting therapy.

The relationship between prognoses of lung cancer patients or lung adenocarcinoma patients and diabetes mellitus was examined.

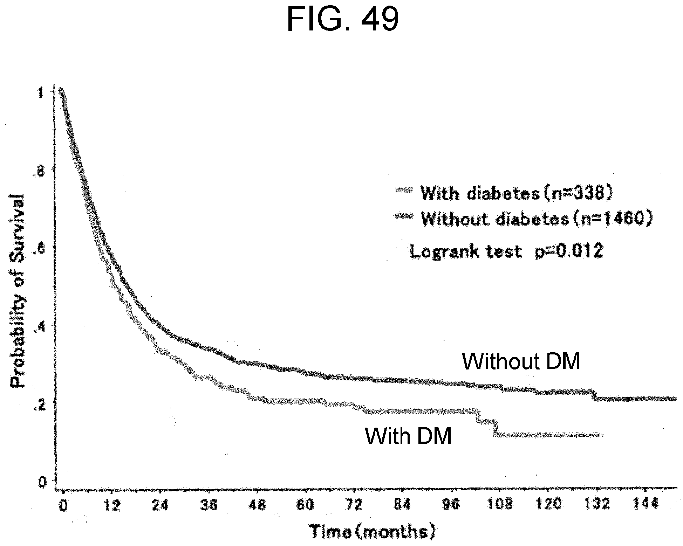

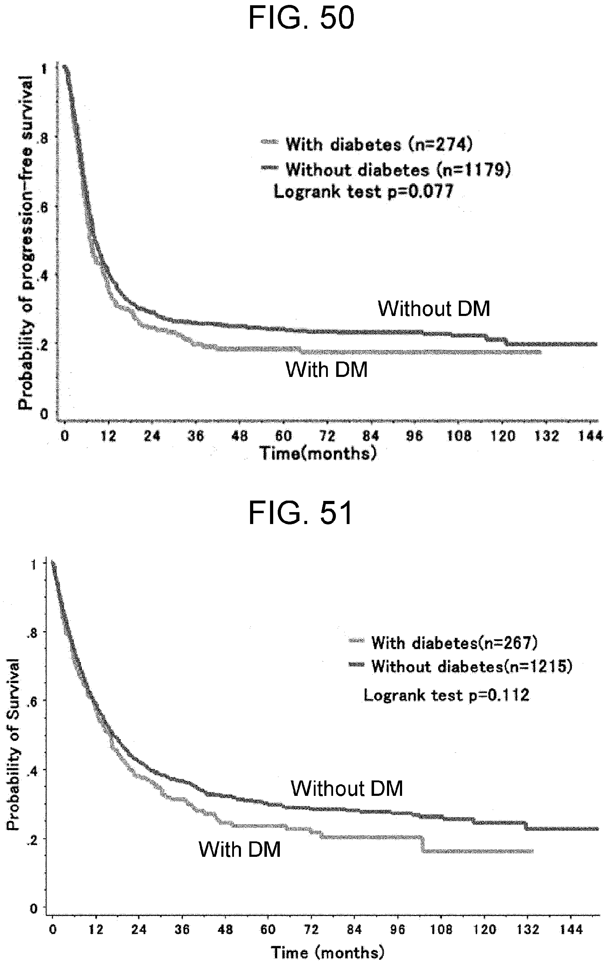

The relationship between the survival rate and the presence or absence of diabetes mellitus for 1798 lung cancer cases was as shown in Figure 49. Lung cancer patients with diabetes mellitus (DM) had poorer prognoses than lung cancer patients without DM. The relationship between the recurrence-free survival time and the presence or absence of diabetes mellitus for the same 1798 lung cancer cases was as shown in Figure 50. The result showed that the presence or absence of DM caused no significant difference in recurrence-free survival time, and revealed a tendency that lung cancer patients with DM had poorer prognoses than lung cancer patients without DM.

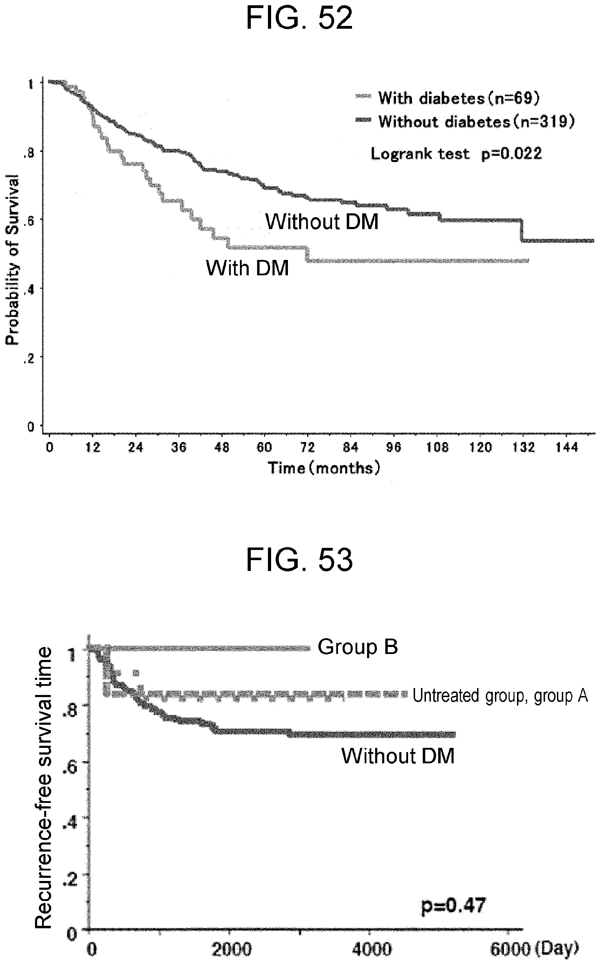

The relationship between the survival rate and the type of diabetes mellitus treatment was examined for 1482 cases of non-small-cell lung cancer patients. Analysis of the 1482 cases of patients found that the presence or absence of DM caused no significant difference in recurrence-free survival time, and revealed a tendency that non-small-cell lung cancer patients with DM had poorer prognoses than non-small-cell lung cancer patients without DM (see Figure 51). In addition, the relationship between the survival rate and the type of diabetes mellitus treatment was examined for patients having been subjected to surgical resection of cancer (388 cases) among the non-small-cell lung cancer patients. The result revealed, as shown in Figure 52, that for the non-small-cell lung cancer patients having been subjected to surgical therapy, patients without diabetes mellitus (DM) had better prognoses than patients with diabetes mellitus.

Next, the relationship between the recurrence-free survival time and the type of diabetes mellitus treatment was examined for 194 cases of lung adenocarcinoma patients. The 194 cases were grouped by the type of treatment into group A (n = 12), group B (n = 3), and an untreated group (n = 173). Group A was a group under treatment with an insulin secretion promoter, and group B was a group under treatment with an insulin resistance improver. The patients of group A were subjected to insulin, Amaryl, or glimepiride treatment. The patients of group B were treated with buformin (Dibetos), metformin (Melbin), pioglitazone (Actos), or pioglitazone/alogliptin (Liovel). For three patients of group A, an insulin resistance improver was used in combination. For one case of group B, a glucose absorption/excretion modulator was used in combination.

The result was as shown in Figure 53. Group B, the patients of which were treated with an insulin resistance improver, had the best prognosis. In contrast to this, no difference in prognoses was found between group A, the patients of which were treated with an insulin secretion promoter, and the untreated group. The result for lung adenocarcinoma showed that the group of patients with diabetes mellitus had better prognoses than the group of patients without diabetes mellitus, and group B, the patients of which were treated with an insulin resistance improver, had the best prognosis.

Those results revealed that lung adenocarcinoma patients with diabetes mellitus had good prognoses, and patients treated with an insulin resistance improver had much better prognoses. This consequence was in contrast to that for all the lung cancer patients and that for all the non-small-cell lung cancer patients. The reason why group B had good prognoses is presumably that insulin does not function well in lung adenocarcinoma patients and the resulting high blood glucose levels allow energy supply to cancer, which is blocked through reduction in blood glucose by improving the function of insulin.

## Claims

1. A method for predicting a prognosis of a cancer patient, the method comprising:
a detection step of detecting expression of SGLT2 protein in a lung adenocarcinoma tissue obtained from a lung adenocarcinoma patient, wherein a case that the adenocarcinoma tissue is negative for expression of SGLT2 indicates that the prognosis is good, and a case that the adenocarcinoma tissue is positive for expression of SGLT2 indicates that the prognosis is poor.

2. The method according to claim 1, wherein the cancer patient is a patient after cancer resection.

3. The method according to claim 1 or 2, wherein whether the adenocarcinoma tissue is positive or negative for the expression is determined on the basis of a ratio of positive cells to cancer cells.

4. The method according to any one of claims 1 to 3, further comprising predicting that the prognosis is good if the patient has a mutation in epidermal growth factor receptor (EGFR) and predicting that the prognosis is poor if the patient has no mutation in EGFR.

5. The method according to any one of claims 1 to 4, wherein the prognosis is based on at least one or more selected from the group consisting of a recurrence rate and a survival rate.

6. The method according to any one of claims 1 to 5, further comprising determining whether the patient is a diabetes mellitus patient or not, wherein
(a) a case that the patient is a diabetes mellitus patient and negative for expression of SGLT2 indicates that the prognosis of the patient is good, or
(b) a case that the patient is a non-diabetes mellitus patient and positive for expression of SGLT2 indicates that the prognosis of the patient is poor.

7. The method according to any one of claims 1 to 6, wherein the lung adenocarcinoma is early-stage cancer selected from the group consisting of Stage I cancer and Stage II cancer.

8. A pharmaceutical composition comprising an SGLT2 inhibitor for use in treating lung adenocarcinoma in an SGLT2-positive patient.

9. The pharmaceutical composition according to claim 8, wherein the patient is an SGLT2-positive diabetes mellitus patient.

10. The pharmaceutical composition according to claim 8, wherein the patient is an SGLT2-positive non-diabetes mellitus patient.

11. A method for predicting a prognosis of a lung adenocarcinoma patient, the method comprising:
a detection step of detecting expression of SGLT1 in a lung adenocarcinoma tissue obtained from a lung adenocarcinoma patient, wherein a case that the adenocarcinoma tissue is positive for expression of SGLT1 indicates that the prognosis is good or possibly good, and a case that the adenocarcinoma tissue is negative for expression of SGLT1 indicates that the prognosis is poor or possibly poor, wherein the method excludes diagnosis.

12. The method according to claim 11, further comprising a detection step of detecting expression of SGLT2 in the lung adenocarcinoma tissue, wherein a case that the adenocarcinoma tissue is positive for expression of SGLT1 and negative for expression of SGLT2 indicates that the prognosis is good or possibly good, and a case that the adenocarcinoma tissue is negative for expression of SGLT1 and positive for expression of SGLT2 indicates that the prognosis is poor or possibly poor.

13. The method according to claim 11 or 12, wherein the adenocarcinoma patient is a patient after cancer resection.

14. The method according to any one of claims 11 to 13, wherein whether the adenocarcinoma tissue is positive or negative for each expression is determined on the basis of a ratio of positive cells to cancer cells.

15. The method according to any one of claims 11 to 14, wherein each expression is expression of protein.

16. The method according to any one of claims 11 to 15, wherein the prognosis is based on at least one or more selected from the group consisting of a recurrence rate and a survival rate.

17. The method according to any one of claims 11 to 16, further comprising determining whether the patient is a diabetes mellitus patient or not, wherein
(a) a case that the patient is a diabetes mellitus patient and positive for expression of SGLT1 indicates that the prognosis of the patient is good or possibly good, and
(b) a case that the patient is a non-diabetes mellitus patient and negative for expression of SGLT1 indicates that the prognosis of the patient is poor or possibly poor.

18. The method according to any one of claims 11 to 17, wherein the lung adenocarcinoma is early-stage cancer selected from the group consisting of Stage I cancer and Stage II cancer.

19. A pharmaceutical composition comprising an insulin resistance improver for use in treating a subject having diabetes mellitus and lung adenocarcinoma.

20. The pharmaceutical composition according to claim 19, wherein the insulin resistance improver is one or more selected from the group consisting of biguanide drugs and thiazolidine drugs.
